(19) Europäisches Patentamt
European Patent Office
Office européen des brevets

(11) EP 3 134 564 B1

(12) **EUROPEAN PATENT SPECIFICATION**

(45) Date of publication and mention of the grant of the patent:
**20.06.2018 Bulletin 2018/25**

(21) Application number: **15722300.9**

(22) Date of filing: **16.04.2015**

(51) Int Cl.:
***D01F 1/10*** *(2006.01)* ***D01F 6/14*** *(2006.01)*
***D01F 6/34*** *(2006.01)* ***A61Q 5/02*** *(2006.01)*
***A61Q 19/00*** *(2006.01)* ***A61K 8/81*** *(2006.01)*
***A61K 8/02*** *(2006.01)* *A61Q 19/10* *(2006.01)*

(86) International application number:
**PCT/US2015/026054**

(87) International publication number:
**WO 2015/164159 (29.10.2015 Gazette 2015/43)**

(54) **FILAMENTS AND FIBROUS STRUCTURES EMPLOYING SAME**

FILAMENTE UND FASERSTRUKTUREN DAMIT

ELÉMENTS FIBREUX ET STRUCTURES FIBREUSES LES EMPLOYANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.04.2014 US 201461982469 P**

(43) Date of publication of application:
**01.03.2017 Bulletin 2017/09**

(73) Proprietor: **The Procter & Gamble Company**
**Cincinnati, OH 45202 (US)**

(72) Inventors:
- **MAO, Min**
  **Cincinnati, OH 45202 (US)**
- **SIVIK, Mark Robert**
  **Cincinnati, OH 45202 (US)**
- **HAMERSKY, Mark William**
  **Cincinnati, OH 45202 (US)**
- **DENOME, Frank William**
  **Cincinnati, OH 45202 (US)**

(74) Representative: **Patent Boutique LLP**
**10A Printing House Yard**
**Hackney Road**
**London E2 7PR (GB)**

(56) References cited:
**WO-A1-2006/008916**
**WO-A2-01/47567**
**CN-A- 101 509 176**
**JP-A- 2000 212 828**
**JP-A- 2010 126 856**
**JP-B2- 4 128 580**
**US-A1- 2012 052 036**

- **PATTAMA TAEPAIBOON ET AL: "Effect of cross-linking on properties and release characteristics of sodium salicylate-loaded electrospun poly(vinyl alcohol) fibre mats; Effect of cross-linking on properties and release characteristics of sodium salicylate-loaded electrospun poly(vinyl alcohol) fibre mats", NANOTECHNOLOGY, IOP, BRISTOL, GB, vol. 18, no. 17, 2 May 2007 (2007-05-02), page 175102, XP020119059, ISSN: 0957-4484, DOI: 10.1088/0957-4484/18/17/175102**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to filaments and more particularly to filaments comprising a vinyl acetate-vinyl alcohol copolymer (a partially hydrolyzed polyvinyl alcohol) and fibrous structures comprising such filaments and methods for making same.

BACKGROUND OF THE INVENTION

Partially hydrolyzed polyvinyl alcohols (vinyl acetate-vinyl alcohol copolymers) exhibit various degrees of hydrolysis. For example, high hydrolysis vinyl acetate-vinyl alcohol copolymers exhibit an average degree of hydrolysis of 87 mol% or greater (87 mol% or greater alcohol units) (commonly referred to as "% hydrolyzed") and low hydrolysis vinyl acetate-vinyl alcohol copolymers exhibit an average degree of hydrolysis of not more than 84 mol% and/or less than 84 mol% and/or less than 82 mol% (84 mol% and/or less than 84 mol% and/or less than 82 mol% alcohol units) (also commonly referred to as "% hydrolyzed").

Filaments comprising high hydrolysis vinyl acetate-vinyl alcohol copolymers, such as Selvol™ 523 (87-89% hydrolyzed, Mw 100,000 g/mol), commercially available from Sekisui Specialty Chemicals, and fibrous structures made therefrom are known in the art. However, one problem with such known filaments and/or fibrous structures is the fact that the high hydrolysis vinyl acetate-vinyl alcohol copolymers exhibit dissolution negatives, such as gel blocking during dissolution, such as during use by a consumer. In other words, upon being subjected to a dissolution condition, such as being contacted with excess water, the viscosity of the high hydrolysis vinyl acetate-vinyl alcohol copolymer from the dissolving filaments and/or fibrous structures significantly increase. In fact, the viscosity often increases to the point of gel blocking, thus inhibiting full dissolution of the filaments and/or fibrous structures. In addition to the dissolution negatives, the high hydrolysis vinyl acetate-vinyl alcohol copolymers also exhibit negatives when present in high solids systems, such as greater than 25% and/or greater than 30% by weight solids, such as filaments that comprise greater than 25% and/or greater than 30% by weight of one or more active agents that are releasable from the filaments upon being exposed to conditions of intended use, such as being exposed to excess water.

Accordingly, there exists a need for filaments comprising vinyl acetate-vinyl alcohol copolymers (partially hydrolyzed polyvinyl alcohols) that overcome the negatives described above and fibrous structures made therefrom and methods for making same.

SUMMARY OF THE INVENTION

The present invention fulfills the needs described above by providing novel filaments and fibrous structures made therefrom and methods for making same.

One solution to the problem described above is a filament comprising a low hydrolysis (84% or less hydrolysis) vinyl acetate-vinyl alcohol copolymer. In particular, the filament of the invention comprises one or more filament-forming materials comprising one or more vinyl acetate-vinyl alcohol copolymers wherein at least one of the vinyl acetate-vinyl alcohol copolymers comprises 84 mol% or less alcohol units; and wherein the filament comprises one or more active agents present within the filament, wherein the least one of active agent is selected from the group consisting of: skin benefit agents, medicinal agents, lotions, fabric care agents, dishwashing agents, carpet care agents, surface care agents, hair care agents, air care agents, and mixtures thereof; and wherein the one or more filament-forming materials and the one or more active agents are present in the filament at a weight ratio of filament-forming materials to active agents of 1.85 or less. The filament and/or fibrous structure made therefrom exhibits improved dissolution over known filaments comprising vinyl acetate-vinyl alcohol copolymers as measured according to the Hand Dissolution Test Method and/or the Dissolution Test Method described herein. It has unexpectedly been found that vinyl acetate-vinyl alcohol copolymers that exhibit an average degree of hydrolysis of 84 mol% or less (84 mol% or less alcohol units) exhibit drastically different properties than vinyl acetate-vinyl alcohol copolymers that exhibit an average degree of hydrolysis of 87 mol% (87 mol% or greater alcohol units) especially with respect to dissolution, for example dissolution of filaments comprising such vinyl acetate-vinyl alcohol copolymers and/or fibrous structures made therefrom.

In one example of the present invention, a filament comprising one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units, is provided.

In another example of the present invention, a filament comprising one or more filament-forming materials comprising one or more low hydrolysis vinyl acetate-vinyl alcohol copolymers, is provided.

In another example of the present invention, a filament comprising one or more filament-forming materials

comprising one or more low hydrolysis vinyl acetate-vinyl alcohol copolymers and void of high hydrolysis vinyl acetate-vinyl alcohol copolymers (for example less than 10% and/or less than 5% and/or less than 3% and/or less than 1% and/or 0% by weight of the high hydrolysis vinyl acetate-vinyl alcohol copolymer), is provided.

In another example of the present invention, a filament comprising one or more filament-forming materials comprising one or more low hydrolysis vinyl acetate-vinyl alcohol copolymers and one or more active agents, for example greater than 10% and/or greater than 20% and/or greater than 40% and/or greater than 50% and/or greater than 60% and/or greater than 80% by weight on a dry filament basis, present within the filament, is provided.

In another example of the present invention, a filament comprising one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units and one or more active agents, for example greater than 10% and/or greater than 20% and/or greater than 40% and/or greater than 50% and/or greater than 60% and/or greater than 80% by weight on a dry filament basis, present within the filament, is provided.

In another example of the present invention, a filament-forming composition suitable for producing filaments of the present invention, for example by a spinning process, the filament-forming composition comprising from about 5% to about 70% by weight of one or more filament-forming materials comprising one or more low hydrolysis vinyl acetate-vinyl alcohol copolymers and from about 95% to about 30% by weight of one or more polar solvents (such as water) is provided.

In another example of the present invention, a filament-forming composition suitable for producing filaments of the present invention, for example by a spinning process, the filament-forming composition comprising from about 5% to about 70% by weight of one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units and from about 95% to about 30% by weight of one or more polar solvents (such as water) is provided.

In yet another example of the present invention, a filament-forming composition suitable for producing filaments of the present invention, for example by a spinning process, the filament-forming composition comprising from about 5% to about 70% by weight of one or more filament-forming materials comprising one or more low hydrolysis vinyl acetate-vinyl alcohol copolymers, from about 5% to about 70% by weight of one or more active agents, and from about 30% to about 70% by weight of one or more polar solvents (such as water) is provided.

In even another example of the present invention, a filament-forming composition suitable for producing filaments of the present invention, for example by a spinning process, the filament-forming composition comprising from about 5% to about 70% by weight of one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units, from about 5% to about 70% by weight of one or more active agents, and from about 30% to about 70% by weight of one or more polar solvents (such as water) is provided.

In still another example of the present invention, a filament-forming composition suitable for producing filaments of the present invention, for example by a spinning process, the filament-forming composition comprising a total level of one or more filament-forming materials comprising one or more low hydrolysis vinyl acetate-vinyl alcohol copolymers, a total level of one or more active agents, and one or more polar solvents (such as water) such that a filament produced from the filament-forming composition comprises 60% or less and/or 50% or less and/or 40% or less and/or 20% or less by weight on a dry filament basis of the one or more filament materials and 40% or greater and/or 50% or greater and/or 60% or greater and/or 80% or greater by weight on a dry filament basis of the one or more active agents and optionally, less than 20% by weight of polar solvents (such as water), is provided. According to the present invention, the one or more filament-forming materials and the one or more active agents are present in the filament at a weight ratio of filament-forming material to active agents of 1.85 or less.

In still another example of the present invention, a filament-forming composition suitable for producing filaments of the present invention, for example by a spinning process, the filament-forming composition comprising a total level of one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units, a total level of one or more active agents, and one or more polar solvents (such as water) such that a filament produced from the filament-forming composition comprises 60% or less and/or 50% or less and/or 40% or less and/or 20% or less by weight on a dry filament basis of the one or more filament materials and 40% or greater and/or 50% or greater and/or 60% or greater and/or 80% or greater by weight on a dry filament basis of the one or more active agents and optionally, less than 20% by weight of polar solvents (such as water), is provided. According to the present invention the one or more filament-forming materials and the one or more active agents are present in the filament at a weight ratio of filament-forming material to active agents of 1.85 or less.

In even still another example of the present invention, a filament comprising one or more filament-forming materials comprising one or more low hydrolysis vinyl acetate-vinyl alcohol copolymers and one or more active agents, for example a mixture of one or more filament-forming materials and one or more active agents, wherein one or more active agents are releasable from the filament when exposed to conditions of intended use, wherein the total level of the one or more filament-forming materials present in the filament is 60% or less and/or 50% or less and/or 40% or less and/or 20% or less by weight on a dry filament basis of the one or more filament materials and the total level of the one or more active agents present in the filament is 40% or greater and/or 50% or greater and/or 60% or greater and/or 80% or greater by weight on a dry filament basis of the one or more active agents, wherein the active agents comprise one or more surfactants, one or more enzymes, one or more suds suppressors, and/or one or more perfumes, is provided.

In even still yet another example of the present invention, a filament comprising one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units and one or more active agents, for example a mixture of one or more filament-forming materials and one or more active agents, wherein one or more active agents are releasable from the filament when exposed to conditions of intended use, wherein the total level of the one or more filament-forming materials present in the filament is 60% or less and/or 50% or less and/or 40% or less and/or 20% or less by weight on a dry filament basis of the one or more filament materials and the total level of the one or more active agents present in the filament is 40% or greater and/or 50% or greater and/or 60% or greater and/or 80% or greater by weight on a dry filament basis of the one or more active agents, wherein the active agents comprise one or more surfactants, one or more enzymes, one or more suds suppressors, and/or one or more perfumes, is provided.

In even still yet another example of the present invention, a fibrous structure (fibrous structure) comprising one or more filaments, for example a plurality of inter-entangled filaments, according to the present invention, is provided.

In yet another example of the present invention, a method for making a filament, the method comprises the steps of:

a. providing a filament-forming composition comprising one or more filament-forming materials comprising one or more low hydrolysis vinyl acetate-vinyl alcohol copolymers and one or more active agents, and optionally, one or more polar solvents (such as water);

b. spinning the filament-forming composition into one or more filaments comprising the one or more filament-forming materials and the one or more active agents, for example that are releasable and/or released from the filament when exposed to conditions of intended use of the filament, wherein the total level of the filament-forming materials present in the filament is 60% or less and/or 50% or less and/or 40% or less and/or 20% or less by weight on a dry filament basis of the one or more filament materials and the total level of the active agents present in the filament is 40% or greater and/or 50% or greater and/or 60% or greater and/or 80% or greater by weight on a dry filament basis of the one or more active agents, is provided.

In even still yet another example of the present invention, a method for making a filament, the method comprises the steps of:

a. providing a filament-forming composition comprising one or more filament-forming materials comprising one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units and one or more active agents, and optionally, one or more polar solvents (such as water);

b. spinning the filament-forming composition into one or more filaments comprising the one or more filament-forming materials and the one or more active agents, for example that are releasable and/or released from the filament when exposed to conditions of intended use of the filament, wherein the total level of the filament-forming materials present in the filament is 60% or less and/or 50% or less and/or 40% or less and/or 20% or less by weight on a dry filament basis of the one or more filament materials and the total level of the active agents present in the filament is 40% or greater and/or 50% or greater and/or 60% or greater and/or 80% or greater by weight on a dry filament basis of the one or more active agents, is provided.

Even though the examples provided herein refer to one or more filaments, fibers made from the filaments of the present invention, such as by cutting a filament of the present invention into fibers, and fibrous structures comprising such fibers, alone or in combination with one or more filaments of the present invention are also within the scope of the present invention.

Accordingly, the present invention provides filaments and/or fibers comprising one or more filament-forming materials comprising one or more low hydrolysis vinyl acetate-vinyl alcohol copolymers and/or one or more vinyl acetate-vinyl alcohol copolymers wherein at least one of the vinyl acetate-vinyl alcohol copolymers comprises 84 mol% or less and/or 82 mol% or less and/or 80 mol% or less and/or greater than 60 mol% or more and/or greater than 70 mol% or more alcohol units, such as active agents, fibrous structures containing such filaments and/or fibers, and a method for making such filaments and/or fibers.

## BRIEF DESCRIPTION OF THE DRAWINGS

Fig. 1 is a schematic representation of an example of a filament according to the present invention;
Fig. 2 is a schematic representation of an example of a fibrous structure according to the present invention;
Fig. 3 is a schematic representation of an apparatus suitable for making a filament according to the present invention;
Fig. 4 is a schematic representation of a die suitable for spinning a filament according to the present invention;
Fig. 5 is a front elevational view of a set-up for the Dissolution Test Method;
Fig. 6 is a partial top view of Fig. 5; and
Fig. 7 is a side elevational view of Fig. 5.

## DETAILED DESCRIPTION OF THE INVENTION

### Definitions

"Filament" as used herein means an elongate particulate having a length greatly exceeding its diameter, i.e. a length to diameter ratio of at least about 10.
The filaments of the present invention may be spun from filament-forming compositions via suitable spinning processes operations, such as meltblowing and/or spunbonding.
The filaments of the present invention may be monocomponent and/or multicomponent. For example, the filaments may comprise bicomponent filaments. The bicomponent filaments may be in any form, such as side-by-side, core and sheath, islands-in-the-sea and the like.
The filaments of the present invention exhibit a length of greater than or equal to 5.08 cm (2 in.) and/or greater than or equal to 7.62 cm (3 in.) and/or greater than or equal to 10.16 cm (4 in.) and/or greater than or equal to 15.24 cm (6 in.).
Filaments are typically considered continuous or substantially continuous in nature. Filaments are relatively longer than fibers (which are less than 5.08 cm in length). Non-limiting examples of filaments include meltblown and/or spunbond filaments. One or more fibers may be formed from a filament of the present invention, such as when the filaments are cut to shorter lengths (such as less than 5.08 cm in length). The present disclosure includes a fiber made from a filament of the present invention, such as a fiber comprising one or more filament-forming materials and one or more additives, such as active agents. Fibers are typically considered discontinuous in nature relative to filaments, which are considered continuous in nature.
"Filament-forming composition" as used herein means a composition that is suitable for making a filament of the present invention such as by meltblowing and/or spunbonding. The filament-forming composition comprises one or more filament-forming materials that exhibit properties that make them suitable for spinning into a filament. In one example, the filament-forming material comprises a polymer. In addition to one or more filament-forming materials, the filament-forming composition may comprise one or more additives, for example one or more active agents. In addition, the filament-forming composition may comprise one or more polar solvents, such as water, into which one or more, for example all, of the filament-forming materials and/or one or more, for example all, of the active agents are dissolved and/or dispersed. As shown in Fig. 1 a filament 10 of the present invention made from a filament-forming composition according to present invention is such that one or more active agents are present in the filament rather than on the filament. The total level of filament-forming materials and total level of active agents present in the filament-forming composition may be any suitable amount so long as the filaments of the present invention are produced therefrom.
In one example, one or more active agents are present in the filament and one or more additional additives, such as active agents, may be present on a surface of the filament. In another example, a filament of the present invention comprises one or more active agents that are present in the filament when originally made, but then bloom to a surface of the filament prior to and/or when exposed to conditions of intended use of the filament.
"Filament-forming material" as used herein means a material, such as a polymer or monomers capable of producing a polymer that exhibits properties suitable for making a filament. The filament-forming material of the invention comprises one or more low hydrolysis, that is, 84% or less hydrolysis, vinyl acetate-vinyl alcohol copolymer. In addition to the one or more low hydrolysis vinyl acetate-vinyl alcohol copolymers, the filament-forming material may further comprise one or more substituted polymers such as an anionic, cationic, zwitterionic, and/or nonionic polymer. In another

example, the polymer may comprise a hydroxyl polymer, such as a polysaccharide, such as starch and/or a starch derivative, such as an ethoxylated starch and/or acid-thinned starch. In another example, the polymer may comprise polyethylenes and/or terephthalates. In yet another example, the filament-forming material is a polar solvent-soluble material. In still another example, the filament-forming material may comprise two or more different filament-forming materials.

As used herein, "vinyl acetate-vinyl alcohol copolymer" refers to a polymer of the following structure (I):

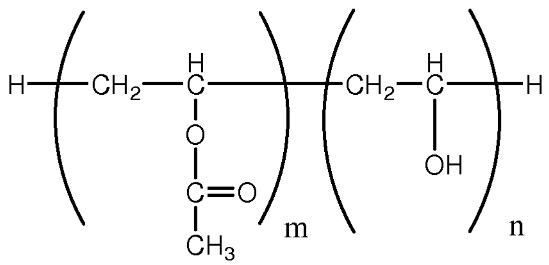

(I)

In structure (I), m and n are integers such that the copolymer has the degree of polymerization and percent alcohol characteristics described herein. For purposes of clarity, this use of the term "copolymer" is intended to convey that the partially hydrolyzed polyvinyl acetate of the present invention comprises vinyl alcohol and vinyl acetate units. As discussed below, the copolymer is routinely prepared by polymerizing vinyl acetate monomer followed by hydrolysis of some of the acetate groups to alcohol groups, as opposed to polymerization of vinyl acetate and vinyl alcohol monomer units (due in-part to the instability of vinyl alcohol).

The filaments and/or fibrous structures of the present invention comprise at least one vinyl acetate-vinyl alcohol copolymer comprising vinyl acetate and vinyl alcohol units, wherein the vinyl acetate-vinyl alcohol copolymer comprises not more than 84% alcohol units. While vinyl acetate-vinyl alcohol copolymers comprised of vinyl acetate and vinyl alcohol units have been used in the past to make good performing dissolvable structures (e.g., US 8,466,099 and US 8,461,090), such vinyl acetate-vinyl alcohol copolymers had a higher degree of vinyl alcohol content (i.e., higher degree of hydrolysis of the polyvinyl acetate starting polymer) - typically around 88% - than the vinyl acetate-vinyl alcohol copolymers of the present invention. One important benefit is the lower alcohol vinyl acetate-vinyl alcohol copolymers allow for the use of significantly less water during production of the filaments and/or fibrous structures of the present invention. Among other things, this allows faster production rates resulting from less water introduction at the front end of the process and reduced drying time and energy after filament and/or fibrous structure formation. These benefits are reflected in the Examples section below.

In one example, the vinyl acetate-vinyl alcohol copolymer comprises not more than about 82.5 mol% alcohol units and in another example not more than about 81 mol% alcohol units. In one example, the vinyl acetate-vinyl alcohol copolymer comprises from about 60 mol% to about 84 mol% alcohol units, in another example from about 65 mol% to about 82.5 mol% alcohol units, and still another example from about 70 mol% to about 81 mol% alcohol units. The percentage of alcohol units (i.e., the degree of hydrolysis) can be determined using standard titration chemistry techniques. One such procedure is described in ISO 15023-2:2003.

The degree of polymerization (weight average molecular weight) of the filament-forming material, for example the vinyl acetate-vinyl alcohol copolymer, is measured using gel permeation chromatography (GPC). This form of chromatography utilizes size exclusion. Separation occurs through a column packed with porous beads. Smaller analytes spend more time in the pores and thus pass through the column more slowly. A detector measures the amount of polymer in the elution solvent as it is eluted. Reference herein to the molecular weight of the copolymer is weighted average molecular weight ("$M_W$"). The $M_W$ of the filament-forming material, for example the vinyl acetate-vinyl alcohol copolymer, can vary widely, but in one example the filament-forming material, for example vinyl acetate-vinyl alcohol copolymer, exhibits a $M_W$ of greater than 10,000 g/mol and/or from about 20,000 g/mol to about 500,000 g/mol, in another example from about 40,000 g/mol to about 400,000 g/mol, in yet another example from about 60,000 g/mol to about 300,000 g/mol, and in still another example from about 70,000 g/mol to about 200,000 g/mol.

In one example, the filaments and/or fibrous structures of the present invention can be prepared by combining two or more vinyl acetate-vinyl alcohol copolymers described herein, wherein the vinyl acetate-vinyl alcohol copolymers differ with respect to either or both of their degree of polymerization and/or their degree of hydrolysis.

The benefits identified can be achieved by using one vinyl acetate-vinyl alcohol copolymer described herein, or it is possible to use two distinct vinyl acetate-vinyl alcohol copolymers.

In one example, the filaments and/or fibrous structures of the present invention can be prepared by combining a vinyl acetate-vinyl alcohol copolymer described herein with a polyvinylalcohol/polyvinylacetate having a higher degree of hydrolysis (e.g., about 88% hydrolyzed; "high hydrolysis polyvinylalcohol"). In such cases, the ratio (weight:weight)

of the vinyl acetate-vinyl alcohol copolymer to high hydrolysis polyvinylalcohol will typically be from about 5:1 to about 1:5. In another example, the filaments and/or fibrous structures of the present invention may be void ("void" meaning less than 10% and/or less than 5% and/or less than 3% and/or less than 1% and/or 0% by weight) of high hydrolysis vinyl acetate-vinyl alcohol copolymers.

The vinyl acetate-vinyl alcohol copolymers useful in the present invention are readily prepared using well known chemistry. One such method is the hydrolysis of a starting polyvinyl ester (polyvinyl acetate; formed via polymerization of vinyl acetate monomer units) of the desired degree of polymerization with absolute alcohols (e.g., methanol) in the presence of catalytic amounts of alkali (e.g., sodium methoxide). In the hydrolysis of polyvinyl acetate to vinyl acetate-vinyl alcohol copolymer, products with different alcohol group contents can be obtained depending on production conditions. Hydrolysis conditions influence the structure of the vinyl acetate-vinyl alcohol copolymer formed. By varying catalyst concentration, reaction temperature, and the reaction time, the content of residual acetyl groups (i.e., unhydrolyzed acetyl groups) can be adjusted routinely. See, for example, Polyvinyl Compounds, Others, Ullmann's Encyclopedia of Industrial Chemistry, Vol. 29, p. 605-609 (2000). Vinyl acetate-vinyl alcohol copolymers are also available commercially, e.g. from Kuraray Europe GmbH.

"Additive" as used herein means any material present in the filament of the present invention that is not a filament-forming material. In one example, an additive comprises an active agent. In another example, an additive comprises a processing aid, for example an extensional aid. In another example, an additive comprises a dissolution aid. In still another example, an additive comprises a filler. In one example, an additive comprises any material present in the filament that its absence from the filament would not result in the filament losing its filament structure, in other words, its absence does not result in the filament losing its solid form. In another example, an additive, for example an active agent, comprises a non-polymer material.

In another example, an additive comprises a plasticizer for the filament. Non-limiting examples of suitable plasticizers for the present invention include polyols, copolyols, polycarboxylic acids, polyesters and dimethicone copolyols. Examples of useful polyols include, but are not limited to, glycerin, diglycerin, propylene glycol, ethylene glycol, butylene glycol, pentylene glycol, cyclohexane dimethanol, hexanediol, 2,2,4-trimethylpentane-1,3-diol, polyethylene glycol (200-600), pentaerythritol, sugar alcohols such as sorbitol, manitol, lactitol and other mono- and polyhydric low molecular weight alcohols (e.g., C2-C8 alcohols); mono di- and oligo-saccharides such as fructose, glucose, sucrose, maltose, lactose, high fructose corn syrup solids, and dextrins, and ascorbic acid.

In one example, the plasticizer includes glycerin and/or propylene glycol and/or glycerol derivatives such as propoxylated glycerol. In still another example, the plasticizer is selected from the group consisting of glycerin, ethylene glycol, polyethylene glycol, propylene glycol, glycidol, urea, sorbitol, xylitol, maltitol, sugars, ethylene bisformamide, amino acids, and mixtures thereof

In another example, an additive comprises a crosslinking agent suitable for crosslinking one or more of the filament-forming materials present in the filaments of the present invention. In one example, the crosslinking agent comprises a crosslinking agent capable of crosslinking hydroxyl polymers together, for example via the hydroxyl polymers hydroxyl moieties. Non-limiting examples of suitable crosslinking agents include imidazolidinones, polycarboxylic acids and mixtures thereof. In one example, the crosslinking agent comprises a urea glyoxal adduct crosslinking agent, for example a dihydroxyimidazolidinone, such as dihydroxyethylene urea ("DHEU"). A crosslinking agent can be present in the filament-forming composition and/or filament of the present invention to control the filament's solubility and/or dissolution in a solvent, such as a polar solvent.

In another example, an additive comprises a rheology modifier, such as a shear modifier and/or an extensional modifier. Non-limiting examples of rheology modifiers include but not limited to polyacrylamide, polyurethanes and polyacrylates that may be used in the filaments of the present invention. Non-limiting examples of rheology modifiers are commercially available from The Dow Chemical Company (Midland, MI).

In yet another example, an additive comprises one or more colors and/or dyes that are incorporated into the filaments of the present invention to provide a visual signal when the filaments are exposed to conditions of intended use and/or when an active agent is released from the filaments and/or when the filament's morphology changes.

In still yet another example, an additive comprises one or more release agents and/or lubricants. Non-limiting examples of suitable release agents and/or lubricants include fatty acids, fatty acid salts, fatty alcohols, fatty esters, sulfonated fatty acid esters, fatty amine acetates, fatty amide, silicones, aminosilicones, fluoropolymers, and mixtures thereof. In one example, the release agents and/or lubricants are applied to the filament, in other words, after the filament is formed. In one example, one or more release agents/lubricants are applied to the filament prior to collecting the filaments on a collection device to form a fibrous structure. In another example, one or more release agents/lubricants are applied to a fibrous structure formed from the filaments of the present invention prior to contacting one or more fibrous structures, such as in a stack of fibrous structures. In yet another example, one or more release agents/lubricants are applied to the filament of the present invention and/or fibrous structure comprising the filament prior to the filament and/or fibrous structure contacting a surface, such as a surface of equipment used in a processing system so as to facilitate removal of the filament and/or fibrous structure and/or to avoid layers of filaments and/or fibrous structures of

the present invention sticking to one another, even inadvertently. In one example, the release agents/lubricants comprise particulates.

In even still yet another example, an additive comprises one or more anti-blocking and/or detackifying agents. Non-limiting examples of suitable anti-blocking and/or detackifying agents include starches, starch derivatives, crosslinked polyvinylpyrrolidone, crosslinked cellulose, microcrystalline cellulose, silica, metallic oxides, calcium carbonate, talc, mica, and mixtures thereof.

"Conditions of intended use" as used herein means the temperature, physical, chemical, and/or mechanical conditions that a filament of the present invention is exposed to when the filament is used for one or more of its designed purposes. For example, if a filament and/or a fibrous structure comprising a filament are designed to be used in a washing machine for laundry care purposes, the conditions of intended use will include those temperature, chemical, physical and/or mechanical conditions present in a washing machine, including any wash water, during a laundry washing operation. In another example, if a filament and/or a fibrous structure comprising a filament are designed to be used by a human as a shampoo for hair care purposes, the conditions of intended use will include those temperature, chemical, physical and/or mechanical conditions present during the shampooing of the human's hair. Likewise, if a filament and/or fibrous structure comprising a filament is designed to be used in a dishwashing operation, by hand or by a dishwashing machine, the conditions of intended use will include the temperature, chemical, physical and/or mechanical conditions present in a dishwashing water and/or dishwashing machine, during the dishwashing operation.

"Active agent" as used herein means an additive that produces an intended effect in an environment external to a filament and/or fibrous structure comprising the filament of the present, such as when the filament is exposed to conditions of intended use of the filament and/or fibrous structure comprising the filament. In one example, an active agent comprises an additive that treats a surface, such as a hard surface (i.e., kitchen countertops, bath tubs, toilets, toilet bowls, sinks, floors, walls, teeth, cars, windows, mirrors, dishes) and/or a soft surface (i.e., fabric, hair, skin, carpet, crops, plants,). In another example, an active agent comprises an additive that creates a chemical reaction (i.e., foaming, fizzing, coloring, warming, cooling, lathering, disinfecting and/or clarifying and/or chlorinating, such as in clarifying water and/or disinfecting water and/or chlorinating water). In yet another example, an active agent comprises an additive that treats an environment (i.e., deodorizes, purifies, perfumes air). In one example, the active agent is formed in situ, such as during the formation of the filament containing the active agent, for example the filament may comprise a water-soluble polymer (e.g., starch) and a surfactant (e.g., anionic surfactant), which may create a polymer complex or coacervate that functions as the active agent used to treat fabric surfaces.

"Treats" as used herein with respect to treating a surface means that the active agent provides a benefit to a surface or environment. Treats includes regulating and/or immediately improving a surface's or environment's appearance, cleanliness, smell, purity and/or feel. In one example treating in reference to treating a keratinous tissue (for example skin and/or hair) surface means regulating and/or immediately improving the keratinous tissue's cosmetic appearance and/or feel. For instance, "regulating skin, hair, or nail (keratinous tissue) condition" includes: thickening of skin, hair, or nails (e.g., building the epidermis and/or dermis and/or sub-dermal [e.g., subcutaneous fat or muscle] layers of the skin, and where applicable the keratinous layers of the nail and hair shaft) to reduce skin, hair, or nail atrophy, increasing the convolution of the dermal-epidermal border (also known as the rete ridges), preventing loss of skin or hair elasticity (loss, damage and/or inactivation of functional skin elastin) such as elastosis, sagging, loss of skin or hair recoil from deformation; melanin or non-melanin change in coloration to the skin, hair, or nails such as under eye circles, blotching (e.g., uneven red coloration due to, e.g., rosacea) (hereinafter referred to as "red blotchiness"), sallowness (pale color), discoloration caused by telangiectasia or spider vessels, and graying hair.

In another example, treating means removing stains and/or odors from fabric articles, such as clothes, towels, linens, and/or hard surfaces, such as countertops and/or dishware including pots and pans.

"Personal care active agent," as used herein, means an active agent that may be applied to mammalian keratinous tissue without undue undesirable effects.

"Keratinous tissue," as used herein, means keratin-containing layers disposed as the outermost protective covering of mammals and includes, but is not limited to, skin, hair, scalp and nails.

"Beauty benefit," as used herein in reference to mammalian keratinous tissue includes, but is not limited to cleansing, sebum inhibition, reducing the oily and/or shiny appearance of skin and/or hair, reducing dryness, itchiness and/or flakiness, reducing skin pore size, exfoliation, desquamation, improving the appearance of the keratinous tissue, conditioning, smoothening, deodorizing skin and/or providing antiperspirant benefits, etc.

"Beauty benefit active agent," as used herein, refers to an active agent that can deliver one or more beauty benefits.

"Skin care active agent" as used herein, means an active agent that when applied to the skin provides a benefit or improvement to the skin. It is to be understood that skin care active agents are useful not only for application to skin, but also to hair, scalp, nails and other mammalian keratinous tissue.

"Hair care active agent" as used herein, means an active agent that when applied to mammalian hair provides a benefit and/or improvement to the hair. Non-limiting examples of benefits and/or improvements to hair include softness,

static control, hair repair, dandruff removal, dandruff resistance, hair coloring, shape retention, hair retention, and hair growth.

"Fabric care active agent" as used herein means an active agent that when applied to fabric provides a benefit and/or improvement to the fabric. Non-limiting examples of benefits and/or improvements to fabric include cleaning (for example by surfactants), stain removal, stain reduction, wrinkle removal, color restoration, static control, wrinkle resistance, permanent press, wear reduction, wear resistance, pill removal, pill resistance, soil removal, soil resistance (including soil release), shape retention, shrinkage reduction, softness, fragrance, anti-bacterial, anti-viral, odor resistance, and odor removal.

"Dishwashing active agent" as used herein means an active agent that when applied to dishware, glassware, pots, pans, utensils, and/or cooking sheets provides a benefit and/or improvement to the dishware, glassware, pots, pans and/or cooking sheets. Non-limiting example of benefits and/or improvements to the dishware, glassware, pots, pans, utensils, and/or cooking sheets include food and/or soil removal, cleaning (for example by surfactants) stain removal, stain reduction, grease removal, water spot removal and/or water spot prevention, shining, and polishing.

"Hard surface active agent" as used herein means an active agent when applied to floors, countertops, sinks, windows, mirrors, showers, baths, and/or toilets provides a benefit and/or improvement to the floors, countertops, sinks, windows, mirrors, showers, baths, and/or toilets. Non-limiting example of benefits and/or improvements to the floors, countertops, sinks, windows, mirrors, showers, baths, and/or toilets include food and/or soil removal, grease removal, water spot removal and/or water spot prevention, shining, and polishing.

"Agricultural active agent" as used herein means an active agent that when applied to crops and/or plants provides a benefit and/or improvement to the crops and/or plants. For example, insecticides, herbicides, fertilizers, drought resistant agents, are non-limiting examples of suitable agricultural active agents that may be present in the filaments of the present invention.

"Ingestible active agent" as used herein means an active agent that is suitable for ingestion and/or consuming by an animal, for example a mammal, such as a human, by way of mouth, nose, eyes, ears, skin pores, rectum, vagina, or other orifice or wound (such as delivering an active agent by wound dressing) in the animal. Non-limiting examples of ingestible active agents include feminine hygiene active agents, baby care active agents, oral care active agents, medicinal active agents, vitamins, dietary active agents (for example delivered in a new food form), pet care active agents, and mixtures thereof.

"Liquid treatment active agent" as used herein means an active agent that when applied to a liquid such as water and/or alcohol, provides a benefit and/or improvement to the liquid. For example, chlorine and/or other swimming pool chemicals are non-limiting examples of suitable liquid treatment active agents. In another example, water clarifying and/or water disinfecting active agents, such as are used in commercial water filtering and/or water treatment technologies such as PUR® are non-limiting examples of suitable liquid treatment active agents that may be present in the filaments of the present invention. Further, oil dispersants and/or oil scavenging agents are non-limiting examples of other suitable liquid treatment active agents.

"Industrial active agent" as used herein means an active agent that provides a benefit within an article of manufacture. For example, glue and/or adhesive to provide bonding between two object, insecticides incorporated into insulation, such as housing insulation, oxygen scavenging active agents incorporated into packaging for food and/or perishable goods, insect repellants incorporated into articles used by humans to repel insects, and moisture scavengers incorporated into desiccants are non-limiting examples of industrial active agents that may be present in the filaments of the present invention.

"Weight ratio" as used herein means the weight of filament-forming material (g or %) on a dry weight basis in the filament to the weight of additive, such as active agent(s) (g or %) on a dry weight basis in the filament.

"Hydroxyl polymer" as used herein includes any hydroxyl-containing polymer that can be incorporated into a filament of the present invention, for example as a filament-forming material. In one example, the hydroxyl polymer of the present invention includes greater than 10% and/or greater than 20% and/or greater than 25% by weight hydroxyl moieties.

"Biodegradable" as used herein means, with respect to a material, such as a filament as a whole and/or a polymer within a filament, such as a filament-forming material, that the filament and/or polymer is capable of undergoing and/or does undergo physical, chemical, thermal and/or biological degradation in a municipal solid waste composting facility such that at least 5% and/or at least 7% and/or at least 10% of the original filament and/or polymer is converted into carbon dioxide after 30 days as measured according to the OECD (1992) Guideline for the Testing of Chemicals 301B; Ready Biodegradability - $CO_2$ Evolution (Modified Sturm Test) Test incorporated herein by reference.

"Non-biodegradable" as used herein means, with respect to a material, such as a filament as a whole and/or a polymer within a filament, such as a filament-forming material, that the filament and/or polymer is not capable of undergoing physical, chemical, thermal and/or biological degradation in a municipal solid waste composting facility such that at least 5% of the original filament and/or polymer is converted into carbon dioxide after 30 days as measured according to the OECD (1992) Guideline for the Testing of Chemicals 301B; Ready Biodegradability - $CO_2$ Evolution

(Modified Sturm Test) Test incorporated herein by reference.

"Non-thermoplastic" as used herein means, with respect to a material, such as a filament as a whole and/or a polymer within a filament, such as a filament-forming material, that the filament and/or polymer exhibits no melting point and/or softening point, which allows it to flow under pressure, in the absence of a plasticizer, such as water, glycerin, sorbitol, urea and the like.

"Non-thermoplastic, biodegradable filament" as used herein means a filament that exhibits the properties of being biodegradable and non-thermoplastic as defined above.

"Non-thermoplastic, non-biodegradable filament" as used herein means a filament that exhibits the properties of being non-biodegradable and non-thermoplastic as defined above.

"Thermoplastic" as used herein means, with respect to a material, such as a filament as a whole and/or a polymer within a filament, such as a filament-forming material, that the filament and/or polymer exhibits a melting point and/or softening point at a certain temperature, which allows it to flow under pressure, in the absence of a plasticizer

"Thermoplastic, biodegradable filament" as used herein means a filament that exhibits the properties of being biodegradable and thermoplastic as defined above.

"Thermoplastic, non-biodegradable filament" as used herein means a filament that exhibits the properties of being non-biodegradable and thermoplastic as defined above.

"Non-cellulose-containing" as used herein means that less than 5% and/or less than 3% and/or less than 1% and/or less than 0.1% and/or 0% by weight of cellulose polymer, cellulose derivative polymer and/or cellulose copolymer is present in filament. In one example, "non-cellulose-containing" means that less than 5% and/or less than 3% and/or less than 1% and/or less than 0.1% and/or 0% by weight of cellulose polymer is present in filament.

"Polar solvent-soluble material" as used herein means a material that is miscible in a polar solvent. In one example, a polar solvent-soluble material is miscible in alcohol and/or water. In other words, a polar solvent-soluble material is a material that is capable of forming a stable (does not phase separate for greater than 5 minutes after forming the homogeneous solution) homogeneous solution with a polar solvent, such as alcohol and/or water at ambient conditions.

"Alcohol-soluble material" as used herein means a material that is miscible in alcohol. In other words, a material that is capable of forming a stable (does not phase separate for greater than 5 minutes after forming the homogeneous solution) homogeneous solution with an alcohol at ambient conditions.

"Water-soluble material" as used herein means a material that is miscible in water. In other words, a material that is capable of forming a stable (does not separate for greater than 5 minutes after forming the homogeneous solution) homogeneous solution with water at ambient conditions.

"Non-polar solvent-soluble material" as used herein means a material that is miscible in a non-polar solvent. In other words, a non-polar solvent-soluble material is a material that is capable of forming a stable (does not phase separate for greater than 5 minutes after forming the homogeneous solution) homogeneous solution with a non-polar solvent.

"Ambient conditions" as used herein means 73°F $\pm$ 4°F (about 23°C $\pm$ 2.2°C) and a relative humidity of 50% $\pm$ 10%.

"Length" as used herein, with respect to a filament, means the length along the longest axis of the filament from one terminus to the other terminus. If a filament has a kink, curl or curves in it, then the length is the length along the entire path of the filament.

"Diameter" as used herein, with respect to a filament, is measured according to the Diameter Test Method described herein. In one example, a filament of the present invention exhibits a diameter of less than 100 $\mu$m and/or less than 75 $\mu$m and/or less than 50 $\mu$m and/or less than 25 $\mu$m and/or less than 20 $\mu$m and/or less than 15 $\mu$m and/or less than 10 $\mu$m and/or less than 6 $\mu$m and/or greater than 1 $\mu$m and/or greater than 3 $\mu$m.

"Triggering condition" as used herein in one example means anything, as an act or event, that serves as a stimulus and initiates or precipitates a change in the filament, such as a loss or altering of the filament's physical structure and/or a release of an additive, such as an active agent. In another example, the triggering condition may be present in an environment, such as water, when a filament and/or fibrous structure of the present invention is added to the water. In other words, nothing changes in the water except for the fact that the filament and/or fibrous structure of the present invention are added to the water.

"Morphology changes" as used herein with respect to a filament's morphology changing means that the filament experiences a change in its physical structure. Non-limiting examples of morphology changes for a filament of the present invention include dissolution, melting, swelling, shrinking, breaking into pieces, exploding, lengthening, shortening, and combinations thereof. The filaments of the present invention may completely or substantially lose their filament physical structure or they may have their morphology changed or they may retain or substantially retain their filament physical structure as they are exposed to conditions of intended use.

"By weight on a dry filament basis" means that the weight of the filament measured immediately after the filament has been conditioned in a conditioned room at a temperature of 73°F $\pm$ 4°F (about 23°C $\pm$ 2.2°C) and a relative

humidity of 50% ± 10% for 2 hours. In one example, "by weight on a dry filament basis" means that the filament comprises less than 20% and/or less than 15% and/or less than 10% and/or less than 7% and/or less than 5% and/or less than 3% and/or to 0% and/or to greater than 0% based on the weight of the filament of moisture, such as water, for example free water, as measured according to the Water Content Test Method described herein.

"Total level" as used herein, for example with respect to the total level of one or more active agents present in the filament, means the sum of the weights or weight percent of all of the subject materials, for example active agents. In other words, a filament may comprise 25% by weight on a dry filament basis of an anionic surfactant, 15% by weight on a dry filament basis of a nonionic surfactant, 10% by weight of a chelant, and 5% of a perfume so that the total level of active agents present in the filament is greater than 50%; namely 55% by weight on a dry filament basis.

"Web" as used herein means a collection of formed fibers and/or filaments, such as a fibrous structure, and/or a sheet formed of fibers and/or filaments, such as continuous filaments, of any nature or origin associated with one another. In one example, the web is a sheet that is formed via a spinning process, not a cast process.

"Fibrous structure" for purposes of the present invention as used herein and as defined generally by European Disposables and Nonwovens Association (EDANA) means a sheet of fibers and/or filaments, such as continuous filaments, of any nature or origin, that have been formed into a web by any means, and may be bonded together by any means, with the exception of weaving or knitting. Felts obtained by wet milling are not fibrous structures. In one example, a fibrous structure according to the present invention means an orderly arrangement of filaments within a structure in order to perform a function. In one example, a fibrous structure of the present invention is an arrangement comprising a plurality of two or more and/or three or more filaments that are inter-entangled or otherwise associated with one another to form a fibrous structure. In one example, the fibrous structure of the present invention may comprise, in addition to the filaments of the present invention, one or more solid additives, such as particulates and/or fibers.

"Particulates" as used herein means granular substances and/or powders.

As used herein, the articles "a" and "an" when used herein, for example, "an anionic surfactant" or "a fiber" is understood to mean one or more of the material that is claimed or described.

All percentages and ratios are calculated by weight unless otherwise indicated. All percentages and ratios are calculated based on the total composition unless otherwise indicated.

Unless otherwise noted, all component or composition levels are in reference to the active level of that component or composition, and are exclusive of impurities, for example, residual solvents or by-products, which may be present in commercially available sources.

## Filament

The filament of the present invention comprises one or more filament-forming materials and one or more active agents, for example a mixture of one or more filament-forming materials and one or more active agents, wherein one or more of the active agents are releasable from the filament, such as when the filament is exposed to conditions of intended use, wherein the total level of the one or more filament-forming materials present in the filament is 60% or less and/or 50% or less and/or 40% or less and/or 20% or less by weight on a dry filament basis and the total level of the one or more active agents present in the filament is greater than 40% and/or greater than 50% and/or greater than 60% and/or greater than 80% by weight on a dry filament basis.

A filament formed from one or more filament-forming materials, with or without one or more additives (non-active agent additives), and then coated or contacted with one or more active agents is not within the scope of the present invention. However, a filament formed from one or more filament-forming materials and one or more active agents, for example a mixture (i.e., a filament-forming composition of the present invention) of one or more filament-forming materials and one or more active agents, with or without one or more non-active agent additives, wherein the filament is then coated with one or more active agents is within the scope of the present invention.

In one example, the filament of the present invention comprises one or more filament-forming materials and one or more active agents wherein the total level of filament-forming materials present in the filament is from about 5% to less than 80% by weight on a dry filament basis and the total level of active agents present in the filament is greater than 20% to about 95% by weight on a dry filament basis.

In one example, the filament of the present invention comprises at least 5% and/or at least 10% and/or at least 15% and/or at least 20% and/or less than 50% and/or less than 45% and/or less than 40% and/or less than 35% and/or less than 30% and/or less than 25% by weight on a dry filament basis of the filament-forming materials and greater than 50% and/or at least 55% and/or at least 60% and/or at least 65% and/or at least 70% and/or less than 95% and/or less than 90% and/or less than 85% and/or less than 80% and/or less than 75% by weight on a dry filament basis of active agents. In one example, the filament of the present invention comprises greater than 80% by weight on a dry filament basis of active agents. The one or more filament-forming materials and one or more active agents are present in the filament at a weight ratio of total level of filament-forming materials to active agents of 1.85 or less and/or 1.5 or less and/or 1.25 or less and/or 1 or less and/or 0.9 or less and/or 0.8 or less and/or less than 0.7 and/or less than 0.5 and/or

less than 0.4 and/or less than 0.3 and/or greater than 0.1 and/or greater than 0.15 and/or greater than 0.2 to less than 1 and/or to less than 0.7.

In another example, the one or more filament-forming materials and one or more active agents are present in the filament at a weight ratio of total level of filament-forming materials to active agents of 1 or less and/or 0.9 or less and/or 0.8 or less and/or less than 0.7 and/or less than 0.5 and/or less than 0.4 and/or less than 0.3 and/or greater than 0.1 and/or greater than 0.15 and/or greater than 0.2 to less than 1 and/or to less than 0.7.

In still another example, the filament of the present invention comprises from about 5% to less than 50% by weight on a dry filament basis of the 84% or less hydrolysis vinyl acetate-vinyl alcohol copolymer filament-forming material and greater than 50% to about 95% by weight on a dry filament basis of an active agent, for example a surfactant, such as an anionic surfactant. The filament may further comprise a plasticizer, such as glycerin and/or pH adjusting agents, such as citric acid.

In yet another example, the filament of the present invention comprises from about 5% to less than 50% by weight on a dry filament basis of the 84% or less hydrolysis vinyl acetate-vinyl alcohol copolymer filament-forming material and greater than 50% to about 95% by weight on a dry filament basis of an active agent, for example a surfactant, such as an anionic surfactant, wherein the weight ratio of filament-forming material to additive is less than 1. The filament may further comprise a plasticizer, such as glycerin and/or pH adjusting agents, such as citric acid.

In still another example of the present invention, the filament of the present invention comprises 0% to about 20% and/or 0% to less than 20% and/or 0% to less than 15% and/or greater than 0% to less than 15% and/or greater than 0% to less than 12% and/or greater than 2% to less than 10% and/or greater than 4% to less than 8% by weight of water as measured according to the Water Content Test Method described herein. In one example, the filament of the present invention comprises from about 5% to about 10% and/or from about 7% to about 10% by weight of water as measured according to the Water Content Test Method described herein.

In still another example of the present invention, the filament of the present invention exhibits a water content of 0% to about 20% and/or 0% to less than 20% and/or 0% to less than 15% and/or greater than 0% to less than 15% and/or greater than 0% to less than 12% and/or greater than 2% to less than 10% and/or greater than 4% to less than 8% by weight of water as measured according to the Water Content Test Method described herein. In one example, the filament of the present invention comprises from about 5% to about 10% and/or from about 7% to about 10% by weight of water as measured according to the Water Content Test Method described herein.

In even another example of the present invention, a filament comprises one or more filament-forming materials and one or more active agents selected from the group consisting of: surfactants, perfumes, enzymes, bleaching agents, builders, chelants, suds suppressors, suds boosters, sensates, dispersants, and mixtures thereof that are releasable and/or released when the filament is exposed to condition of intended use. In one example, the filament comprises a total level of filament-forming materials of less than 95% and/or less than 90% and/or less than 80% and/or less than 50% and/or less than 35% and/or to about 5% and/or to about 10% and/or to about 20% by weight on a dry filament basis and a total level of active agents selected from the group consisting of: surfactants, perfumes, enzymes, bleaching agents, builders, chelants, suds suppressors, suds boosters, sensates, dispersants, and mixtures thereof of greater than 35% and/or greater than 50% and/or greater than 65% and/or to about 95% and/or to about 90% and/or to about 80% by weight on a dry filament basis. In one example, the active agent comprises one or more enzymes. In another example, the active agent comprises one or more bleaching agents. In yet another example, the active agent comprises one or more builders. In still another example, the active agent comprises one or more chelants.

In yet another example of the present invention, the filaments of the present invention may comprise active agents that may create health and/or safety concerns if they become airborne. For example, the filament may be used to inhibit enzymes within the filament from becoming airborne.

In one example, the filaments of the present invention may be meltblown filaments. In another example, the filaments of the present invention may be spunbond filaments. In another example, the filaments may be hollow filaments prior to and/or after release of one or more of its active agents.

The filaments of the present invention may be hydrophilic or hydrophobic. The filaments may be surface treated and/or internally treated to change the inherent hydrophilic or hydrophobic properties of the filament.

In one example, the filament exhibits a diameter of less than 100 μm and/or less than 75 μm and/or less than 50 μm and/or less than 25 μm and/or less than 10 μm and/or less than 5 μm and/or less than 1 μm as measured according to the Diameter Test Method described herein. In another example, the filament of the present invention exhibits a diameter of greater than 1 μm as measured according to the Diameter Test Method described herein. The diameter of a filament of the present invention may be used to control the rate of release of one or more active agents present in the filament and/or the rate of loss and/or altering of the filament's physical structure.

The filament may comprise two or more different active agents. In one example, the filament comprises two or more different active agents, wherein the two or more different active agents are compatible with one another. In another example, the filament comprises two or more different active agents, wherein the two or more different active agents are incompatible with one another.

In one example, the filament may comprise an active agent within the filament and an active agent on an external surface of the filament, such as coating composition on the filament and/or fibrous structure comprising such filaments. The active agent on the external surface of the filament may be the same or different from the active agent present in the filament. If different, the active agents may be compatible or incompatible with one another.

In one example, a filament of the present invention is preservative free, which means for purposes of the present invention that it contains less than 2% and/or less than 1% and/or less than 0.5% and/or less than 0.25% and/or 0% by weight on a dry filament basis of a preservative.

In one example, one or more active agents may be uniformly distributed or substantially uniformly distributed throughout the filament. In another example, one or more active agents may be distributed as discrete regions within the filament. In still another example, at least one active agent is distributed uniformly or substantially uniformly throughout the filament and at least one other active agent is distributed as one or more discrete regions within the filament. In still yet another example, at least one active agent is distributed as one or more discrete regions within the filament and at least another active agent is distributed as one or more discrete regions different from the first discrete regions within the filament.

The filaments may be used as discrete articles. In one example, the filaments may be applied to and/or deposited on a carrier substrate, for example a wipe, paper towel, bath tissue, facial tissue, sanitary napkin, tampon, diaper, adult incontinence article, washcloth, dryer sheet, laundry sheet, laundry bar, dry cleaning sheet, netting, filter paper, fabrics, clothes, undergarments, and the like.

## Filament-forming Material

At least one of the filament-forming materials present in the filaments of the present invention comprises a low hydrolysis vinyl acetate-vinyl alcohol copolymer, that is, a vinyl acetate-vinyl alcohol copolymer wherein the vinyl acetate-vinyl alcohol copolymer comprises 84 mol% or less and/or 82 mol% or less and/or 80 mol% or less and/or 60 mol% or more and/or 70% or more alcohol units. Additional filament-forming materials may be present in the filaments of the present invention, such as a polymer or monomers capable of producing a polymer that exhibits properties suitable for making a filament, such as by a spinning process. Non-limiting examples of other suitable filament-forming materials are disclosed in U.S. Patent Application Publication No. 2012/0052036. In one example, in addition to the vinyl acetate-vinyl alcohol copolymer(s) of the present invention, the filament-forming materials may further comprise a polar solvent-soluble material, such as an alcohol-soluble material and/or a water-soluble material.

In another example, in addition to the vinyl acetate-vinyl alcohol copolymer(s) of the present invention, the filament-forming materials may further comprise a non-polar solvent-soluble material.

In still another example, in addition to the vinyl acetate-vinyl alcohol copolymer(s) of the present invention, the filament forming materials may further comprise a polar solvent-soluble material and be free (less than 5% and/or less than 3% and/or less than 1% and/or 0% by weight on a dry filament basis) of non-polar solvent-soluble materials.

In still yet another example, the filament-forming materials may be synthetic or of natural origin and it may be chemically, enzymatically, and/or physically modified.

In even another example of the present invention, in addition to the vinyl acetate-vinyl alcohol copolymer(s) of the present invention, the filament-forming material may further comprise a polymer selected from the group consisting of: polymers derived from acrylic monomers such as the ethylenically unsaturated carboxylic monomers and ethylenically unsaturated monomers, other polyvinyl alcohols, polyacrylates, polymethacrylates, copolymers of acrylic acid and methyl acrylate, polyvinylpyrrolidones, polyalkylene oxides, starch and starch derivatives, pullulan, gelatin, hydroxypropylmethylcelluloses, methycelluloses, and carboxymethycelluloses.

In still another example, in addition to the vinyl acetate-vinyl alcohol copolymer(s) of the present invention, the filament-forming material may comprise a polymer selected from the group consisting of: other polyvinyl alcohols, other polyvinyl alcohol derivatives, starch, starch derivatives, cellulose derivatives, hemicellulose, hemicellulose derivatives, proteins, sodium alginate, hydroxypropyl methylcellulose, chitosan, chitosan derivatives, polyethylene glycol, tetramethylene ether glycol, polyvinyl pyrrolidone, hydroxymethyl cellulose, hydroxyethyl cellulose, and mixtures thereof.

In another example, in addition to the vinyl acetate-vinyl alcohol copolymer(s) of the present invention, the filament-forming material may further comprise a polymer selected from the group consisting of: pullulan, hydroxypropylmethyl cellulose, hydroxyethyl cellulose, hydroxypropyl cellulose, polyvinyl pyrrolidone, carboxymethyl cellulose, sodium alginate, xanthan gum, tragacanth gum, guar gum, acacia gum, Arabic gum, polyacrylic acid, methylmethacrylate copolymer, carboxyvinyl polymer, dextrin, pectin, chitin, levan, elsinan, collagen, gelatin, zein, gluten, soy protein, casein, other polyvinyl alcohols, starch, starch derivatives, hemicellulose, hemicellulose derivatives, proteins, chitosan, chitosan derivatives, polyethylene glycol, tetramethylene ether glycol, hydroxymethyl cellulose, and mixtures thereof.

Active Agents

Active agents are a class of additives that are designed and intended to provide a benefit to something other than the filament itself, such as providing a benefit to an environment external to the filament. Active agents may be any suitable additive that produces an intended effect under intended use conditions of the filament. For example, the active agent may be selected from the group consisting of: personal cleansing and/or conditioning agents such as hair care agents such as shampoo agents and/or hair colorant agents, hair conditioning agents, skin care agents, sunscreen agents, and skin conditioning agents; laundry care and/or conditioning agents such as fabric care agents, fabric conditioning agents, fabric softening agents, fabric anti-wrinkling agents, fabric care anti-static agents, fabric care stain removal agents, soil release agents, dispersing agents, suds suppressing agents, suds boosting agents, anti-foam agents, and fabric refreshing agents; hard surface care agents, and/or conditioning agents such as liquid and/or powder dishwashing agents (for hand dishwashing and/or automatic dishwashing machine applications), and polishing agents; other cleaning and/or conditioning agents such as antimicrobial agents, perfume, bleaching agents (such as oxygen bleaching agents, hydrogen peroxide, percarbonate bleaching agents, perborate bleaching agents, chlorine bleaching agents), bleach activating agents, chelating agents, builders, lotions, brightening agents, air care agents, carpet care agents, dye transfer-inhibiting agents, water-softening agents, water-hardening agents, pH adjusting agents, enzymes, flocculating agents, effervescent agents, preservatives, cosmetic agents, make-up removal agents, lathering agents, deposition aid agents, coacervate-forming agents, clays, thickening agents, latexes, silicas, drying agents, odor control agents, antiperspirant agents, cooling agents, warming agents, absorbent gel agents, anti-inflammatory agents, dyes, pigments, acids, and bases; liquid treatment active agents; agricultural active agents; industrial active agents; ingestible active agents such as medicinal agents, teeth whitening agents, tooth care agents, mouthwash agents, periodontal gum care agents, edible agents, dietary agents, vitamins, minerals; water-treatment agents such as water clarifying and/or water disinfecting agents, and mixtures thereof. In particular, according to present invention, at least one of the active agents is selected from the group consisting of: skin benefit agents, medicinal agents, lotions, fabric care agents, dishwashing agents, carpet care agents, surface care agents, hair care agents, air care agents, and mixtures thereof.

Non-limiting examples of suitable cosmetic agents, skin care agents, skin conditioning agents, hair care agents, and hair conditioning agents are described in CTFA Cosmetic Ingredient Handbook, Second Edition, The Cosmetic, Toiletries, and Fragrance Association, Inc. 1988, 1992.

One or more classes of chemicals may be useful for one or more of the active agents listed above. For example, surfactants may be used for any number of the active agents described above. Likewise, bleaching agents may be used for fabric care, hard surface cleaning, dishwashing and even teeth whitening. Therefore, one of ordinary skill in the art will appreciate that the active agents will be selected based upon the desired intended use of the filament and/or fibrous structure made therefrom.

For example, if the filament of the present invention and/or fibrous structure made therefrom is to be used for hair care and/or conditioning then one or more suitable surfactants, such as a lathering surfactant could be selected to provide the desired benefit to a consumer when exposed to conditions of intended use of the filament and/or fibrous structure incorporating the filament.

In one example, if the filament of the present invention and/or fibrous structure made therefrom is designed or intended to be used for laundering clothes in a laundry operation, then one or more suitable surfactants and/or enzymes and/or builders and/or perfumes and/or suds suppressors and/or bleaching agents could be selected to provide the desired benefit to a consumer when exposed to conditions of intended use of the filament and/or fibrous structure incorporating the filament. In another example, if the filament of the present invention and/or fibrous structure made therefrom is designed to be used for laundering clothes in a laundry operation and/or cleaning dishes in a dishwashing operation, then the filament may comprise a laundry detergent composition or dishwashing detergent composition.

In one example, the active agent comprises a non-perfume active agent. In another example, the active agent comprises a non-surfactant active agent. In still another example, the active agent comprises a non-ingestible active agent, in other words an active agent other than an ingestible active agent.

Surfactants

Non-limiting examples of suitable surfactants include anionic surfactants, cationic surfactants, nonionic surfactants, zwitterionic surfactants, amphoteric surfactants, and mixtures thereof. Co-surfactants may also be included in the filaments. For filaments designed for use as laundry detergents and/or dishwashing detergents, the total level of surfactants should be sufficient to provide cleaning including stain and/or odor removal, and generally ranges from about 0.5% to about 95%. Further, surfactant systems comprising two or more surfactants that are designed for use in filaments for laundry detergents and/or dishwashing detergents may include all-anionic surfactant systems, mixed-type surfactant systems comprising anionic-nonionic surfactant mixtures, or nonionic-cationic surfactant mixtures.

The surfactants herein can be linear or branched. In one example, suitable linear surfactants include those

derived from agrochemical oils such as coconut oil, palm kernel oil, soybean oil, or other vegetable-based oils.

Non-limiting examples of other suitable active agents are disclosed in U.S. Patent Application Publication No. 2012/0052036. Further non-limiting examples of surfactants suitable for inclusion in the filaments and/or fibrous structures of the present invention are described below.

The filaments and/or fibrous structures of the present invention may comprise one or more surfactants suitable for application to the hair or skin. Surfactants suitable for use in the filaments and/or fibrous structures of the present invention include anionic surfactants, nonionic surfactants, cationic surfactants, zwitterionic surfactants, amphoteric surfactants, polymeric surfactants or combinations thereof. Although representative surfactants are described herein, the skilled artisan will recognize that other surfactants can be readily substituted and similar benefits can be derived from use of the vinyl acetate-vinyl alcohol copolymers described herein. Each patent described throughout this application is incorporated herein by reference to the extent each provides guidance regarding surfactants suitable for inclusion in the filaments and/or fibrous structures of the present invention.

In one example, the filaments and/or fibrous structures of the present invention may be a lathering personal care product (dried) and may comprise from about 23 wt% to about 75 wt% surfactant, in another example from about 30 wt% to about 70 wt% surfactant, in still another example from about 40 wt% to about 65 wt% surfactant.

Suitable anionic surfactants include alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278. Exemplary anionic surfactants include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. In one embodiment, the anionic surfactant is sodium lauryl sulfate or sodium laureth sulfate.

In one example, the anionic surfactant comprises at least one branched sulfate having the formula $CH_3-(CH_2)_z-CH(R^1)-CH_2-O-(CH_2CH(R^2)O)_y-SO_3M$; where z is from about 3 to about 14; $R^1$ represents H or a hydrocarbon radical comprising 1 to 4 carbon atoms, $R^2$ is H or $CH_3$; $R^1$ and $R^2$ are not both H; y is 0 to about 7; the average value of y is about 1 when y is not = 0; and M is a mono-valent or di-valent, positively-charged cation. Examples of mono-valent positively charged cations include ammonium, sodium, potassium, triethanolamine cation, and examples of di-valent positively charged cations include magnesium. For the foregoing branched sulfates, "average value" means that whereas the composition may comprise molecules having a value of y of other than 1, the average value of y all molecules in the composition is about 1.

Suitable amphoteric or zwitterionic surfactants include those which are known for use in shampoo or other cleansing products. Non limiting examples of suitable zwitterionic or amphoteric surfactants are described in U.S. Patent Nos. 5,104,646 and 5,106,609. Suitable amphoteric surfactants include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic group such as carboxy, sulfonate, sulfate, phosphate, or phosphonate. Exemplary amphoteric detersive surfactants include cocoamphoacetate, cocoamphodiacetate, lauroamphoacetate, lauroamphodiacetate, and mixtures thereof.

Suitable zwitterionic surfactants include those surfactants broadly described as derivatives of aliphatic quaternaryammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. In another embodiment, zwitterionics such as betaines are selected.

Non limiting examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Patent Nos. 3,929,678, 2,658,072; 2,438,091; 2,528,378. In another example, the filaments and/or fibrous structures of the present invention may be a substantially non-lathering personal care product and comprises a) from about 0 wt% to about 10 wt% of an ionic (anionic, zwitterionic, cationic and mixtures thereof) surfactant, in another example from about 0 wt% to about 5 wt% of an ionic surfactant, and in still another example from about 0 wt% to about 2.5 wt% anionic surfactant, and b) from about 1 wt% to about 50 wt% of a nonionic or polymeric surfactant, in another example from about 5 wt% to about 45 wt% of a nonionic or polymeric surfactant, and in still another example from about 10 wt% to about 40 wt% of a nonionic or polymeric surfactant, and combinations thereof.

Suitable nonionic surfactants for use in the present invention include those described in McCutcheon's Detergents and Emulsifiers, North American edition (2010), Allured Publishing Corp., and McCutcheon's Functional Materials, North American edition (2010). Suitable nonionic surfactants for use in the filaments and/or fibrous structures of the present invention include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl glycosides, alkyl polyglucosides, alkylamine oxides, and polyoxyethylenated silicones.

In another example, the nonionic surfactant may be selected from sorbitan esters and alkoxylated derivatives of sorbitan esters including sorbitan monolaurate (SPAN® 20), sorbitan monopalmitate (SPAN® 40), sorbitan monostearate (SPAN® 60), sorbitan tristearate (SPAN® 65), sorbitan monooleate (SPAN® 80), sorbitan trioleate (SPAN® 85), sorbitan isostearate, polyoxyethylene (20) sorbitan monolaurate (Tween® 20), polyoxyethylene (20) sorbitan monopalmitate (Tween® 40), polyoxyethylene (20) sorbitan monostearate (Tween® 60), polyoxyethylene (20) sorbitan monooleate (Tween® 80), polyoxyethylene (4) sorbitan monolaurate (Tween® 21), polyoxyethylene (4) sorbitan monostearate (Tween® 61), polyoxyethylene (5) sorbitan monooleate (Tween® 81), all available from Uniqema, and combinations thereof.

Suitable polymeric surfactants include, but are not limited to, block copolymers of ethylene oxide and fatty alkyl residues, block copolymers of ethylene oxide and propylene oxide, hydrophobically modified polyacrylates, hydrophobically modified celluloses, silicone polyethers, silicone copolyol esters, diquaternary polydimethylsiloxanes, and co-modified amino/polyether silicones.

## Release of Active Agent

One or more active agents may be released from the filament when the filament is exposed to a triggering condition. In one example, one or more active agents may be released from the filament or a part of the filament when the filament or the part of the filament loses its identity, in other words, loses its physical structure. For example, a filament loses its physical structure when the filament-forming material dissolves, melts or undergoes some other transformative step such that the filament structure is lost. In one example, the one or more active agents are released from the filament when the filament's morphology changes.

In another example, one or more active agents may be released from the filament or a part of the filament when the filament or the part of the filament alters its identity, in other words, alters its physical structure rather than loses its physical structure. For example, a filament alters its physical structure when the filament-forming material swells, shrinks, lenthens, and/or shortens, but retains its filament-forming properties.

In another example, one or more active agents may be released from the filament with the filament's morphology not changing (not losing or altering its physical structure).

In one example, the filament may release an active agent upon the filament being exposed to a triggering condition that results in the release of the active agent, such as by causing the filament to lose or alter its identity as discussed above. Non-limiting examples of triggering conditions include exposing the filament to solvent, a polar solvent, such as alcohol and/or water, and/or a non-polar solvent, which may be sequential, depending upon whether the filament-forming material comprises a polar solvent-soluble material and/or a non-polar solvent-soluble material; exposing the filament to heat, such as to a temperature of greater than 75°F and/or greater than 100°F and/or greater than 150°F and/or greater than 200°F and/or greater than 212°F; exposing the filament to cold, such as to a temperature of less than 40°F and/or less than 32°F and/or less than 0°F; exposing the filament to a force, such as a stretching force applied by a consumer using the filament; and/or exposing the filament to a chemical reaction; exposing the filament to a condition that results in a phase change; exposing the filament to a pH change and/or a pressure change and/or temperature change; exposing the filament to one or more chemicals that result in the filament releasing one or more of its active agents; exposing the filament to ultrasonics; exposing the filament to light and/or certain wavelengths; exposing the filament to a different ionic strength; and/or exposing the filament to an active agent released from another filament.

In one example, one or more active agents may be released from the filaments of the present invention when a fibrous structure comprising the filaments is subjected to a triggering step selected from the group consisting of: pre-treating stains on a fabric article with the fibrous structure; forming a wash liquour by contacting the fibrous structure with water; tumbling the fibrous structure in a dryer; heating the fibrous structure in a dryer; and combinations thereof.

## Filament-forming Composition

The filaments of the present invention are made from a filament-forming composition of the present invention. The filament-forming composition may be a polar-solvent-based composition. In one example, the filament-forming composition is an aqueous composition comprising one or more filament-forming materials at least one of which is a low hydrolysis vinyl acetate-vinyl alcohol copolymer, that is, a vinyl acetate-vinyl alcohol wherein the vinyl acetate-vinyl

alcohol comprises 84% or less by weight alcohol units, one or more active agents (such as a surfactant) and optionally a polar solvent (such as water).

The filament-forming composition of the present invention may have a shear viscosity as measured according to the Shear Viscosity Test Method described herein of from about 1 Pascal·Seconds to about 25 Pascal·Seconds and/or from about 2 Pascal·Seconds to about 20 Pascal·Seconds and/or from about 3 Pascal·Seconds to about 10 Pascal·Seconds, as measured at a shear rate of 3,000 $sec^{-1}$ and at the processing temperature (50°C to 100°C).

The filament-forming composition may be processed at a temperature of from about 50°C to about 100°C and/or from about 65°C to about 95°C and/or from about 70°C to about 90°C when making filaments from the filament-forming composition.

In one example, the filament-forming composition may comprise at least 20% and/or at least 30% and/or at least 40% and/or at least 45% and/or at least 50% to about 90% and/or to about 85% and/or to about 80% and/or to about 75% by weight of one or more filament-forming materials, one or more active agents, and mixtures thereof. The filament-forming composition may comprise from about 10% to about 80% by weight of a polar solvent, such as water.

The filament-forming composition may exhibit a Capillary Number of at least 1 and/or at least 3 and/or at least 5 such that the filament-forming composition can be effectively polymer processed into a hydroxyl polymer fiber.

The Capillary number is a dimensionless number used to characterize the likelihood of this droplet breakup. A larger capillary number indicates greater fluid stability upon exiting the die. The Capillary number is defined as follows:

$$Ca = \frac{V * \eta}{\sigma}$$

V is the fluid velocity at the die exit (units of Length per Time),
$\eta$ is the fluid viscosity at the conditions of the die (units of Mass per Length*Time),
$\sigma$ is the surface tension of the fluid (units of mass per $Time^2$). When velocity, viscosity, and surface tension are expressed in a set of consistent units, the resulting Capillary number will have no units of its own; the individual units will cancel out.

The Capillary number is defined for the conditions at the exit of the die. The fluid velocity is the average velocity of the fluid passing through the die opening. The average velocity is defined as follows:

$$V = \frac{Vol'}{Area}$$

Vol' = volumetric flowrate (units of $Length^3$ per Time),
Area = cross-sectional area of the die exit (units of $Length^2$).

When the die opening is a circular hole, then the fluid velocity can be defined as

$$V = \frac{Vol'}{\pi * R^2}$$

R is the radius of the circular hole (units of length).

The fluid viscosity will depend on the temperature and may depend of the shear rate. The definition of a shear thinning fluid includes a dependence on the shear rate. The surface tension will depend on the makeup of the fluid and the temperature of the fluid.

In a fiber spinning process, the filaments need to have initial stability as they leave the die. The Capillary number is used to characterize this initial stability criterion. At the conditions of the die, the Capillary number should be greater than 1 and/or greater than 4.

In one example, the filament-forming composition exhibits a Capillary Number of from at least 1 to about 50 and/or at least 3 to about 50 and/or at least 5 to about 30.

The filament-forming composition of the present invention may have a shear viscosity of from about 1 Pascal·Seconds to about 25 Pascal·Seconds and/or from about 2 Pascal·Seconds to about 20 Pascal·Seconds and/or from about 3 Pascal·Seconds to about 10 Pascal·Seconds, as measured at a shear rate of 3,000 $sec^{-1}$ and at the processing temperature (50°C to 100°C).

The filament-forming composition may be processed at a temperature of from about 50°C to about 100°C

and/or from about 65°C to about 95°C and/or from about 70°C to about 90°C when making fibers from the filament-forming composition.

In one example, the non-volatile components of the spinning composition may comprise from about 20% and/or 30% and/or 40% and/or 45% and/or 50% to about 75% and/or 80% and/or 85% and/or 90%. The non-volatile components may be composed of filament-forming materials, such as backbone polymers, actives and combinations thereof. The volatile component of the spinning composition will comprise the remaining percentage and range from 10% to 80%.

In one example, the filament-forming composition may comprise one or more release agents and/or lubricants. Non-limiting examples of suitable release agents and/or lubricants include fatty acids, fatty acid salts, fatty alcohols, fatty esters, sulfonated fatty acid esters, fatty amine acetates and fatty amides, silicones, aminosilicones, fluoropolymers and mixtures thereof.

In one example, the filament-forming composition may comprise one or more antiblocking and/or detackifying agents. Non-limiting examples of suitable antiblocking and/or detackifying agents include starches, modified starches, crosslinked polyvinylpyrrolidone, crosslinked cellulose, microcrystalline cellulose, silica, metallic oxides, calcium carbonate, talc and mica.

Active agents of the present invention may be added to the filament-forming composition prior to and/or during filament formation and/or may be added to the filament after filament formation. For example, a perfume active agent may be applied to the filament and/or fibrous structure comprising the filament after the filament and/or fibrous structure according to the present invention are formed. In another example, an enzyme active agent may be applied to the filament and/or fibrous structure comprising the filament after the filament and/or fibrous structure according to the present invention are formed. In still another example, one or more particulate active agents, such as one or more ingestible active agents, such as bismuth subsalicylate, which may not be suitable for passing through the spinning process for making the filament, may be applied to the filament and/or fibrous structure comprising the filament after the filament and/or fibrous structure according to the present invention are formed.

## Extensional Aids

In one example, the filament comprises an extensional aid. Non-limiting examples of extensional aids can include polymers, other extensional aids, and combinations thereof.

In one example, the extensional aids have a weight average molecular weight of at least about 500,000 g/mol. In another example, the weight average molecular weight of the extensional aid is from about 500,000 g/mol to about 25,000,000 g/mol, in another example from about 800,000 g/mol to about 22,000,000 g/mol, in yet another example from about 1,000,000 g/mol to about 20,000,000 g/mol, and in another example from about 2,000,000 g/mol to about 15,000,000 g/mol. The high molecular weight extensional aids are preferred in some examples of the invention due to the ability to increase extensional melt viscosity and reducing melt fracture.

The extensional aid, when used in a meltblowing process, is added to the composition of the present invention in an amount effective to visibly reduce the melt fracture and capillary breakage of fibers during the spinning process such that substantially continuous fibers having relatively consistent diameter can be melt spun. Regardless of the process employed to produce filaments, the extensional aids, when used, can be present from about 0.001% to about 10%, by weight on a dry filament basis, in one example, and in another example from about 0.005 to about 5%, by weight on a dry filament basis, in yet another example from about 0.01 to about 1%, by weight on a dry filament basis, and in another example from about 0.05% to about 0.5%, by weight on a dry filament basis.

Non-limiting examples of polymers that can be used as extensional aids can include alginates, carrageenans, pectin, chitin, guar gum, xanthum gum, agar, gum arabic, karaya gum, tragacanth gum, locust bean gum, alkylcellulose, hydroxyalkylcellulose, carboxyalkylcellulose, and mixtures thereof.

Nonlimiting examples of other extensional aids can include carboxyl modified polyacrylamide, polyacrylic acid, polymethacrylic acid, polyvinyl alcohol, polyvinylacetate, polyvinylpyrrolidone, polyethylene vinyl acetate, polyethyleneimine, polyamides, polyalkylene oxides including polyethylene oxide, polypropylene oxide, polyethylenepropylene oxide, and mixtures thereof.

## Method for Making Filament

The filaments of the present invention may be made by any suitable process. A non-limiting example of a suitable process for making the filaments is described below.

In one example, a method for making a filament according to the present invention comprises the steps of:

a. providing a filament-forming composition comprising one or more filament-forming materials and one or more active agents; and

b. spinning the filament-forming composition into one or more filaments comprising the one or more filament-forming

materials and the one or more active agents that are releasable from the filament when exposed to conditions of intended use, wherein the total level of the one or more filament-forming materials present in the filament is 50% or less by weight on a dry filament basis and the total level of the one or more active agents present in the filament is 50% or greater by weight on a dry filament basis.

In one example, during the spinning step, any volatile solvent, such as water, present in the filament-forming composition is removed, such as by drying, as the filament is formed. In one example, greater than 30% and/or greater than 40% and/or greater than 50% of the weight of the filament-forming composition's volatile solvent, such as water, is removed during the spinning step, such as by drying the filament being produced.

The filament-forming composition may comprise any suitable total level of filament-forming materials and any suitable level of active agents so long as the filament produced from the filament-forming composition comprises a total level of filament-forming materials in the filament of from about 5% to 50% or less by weight on a dry filament basis and a total level of active agents in the filament of from 50% to about 95% by weight on a dry filament basis.

In one example, the filament-forming composition may comprise any suitable total level of filament-forming materials and any suitable level of active agents so long as the filament produced from the filament-forming composition comprises a total level of filament-forming materials in the filament of from about 5% to 50% or less by weight on a dry filament basis and a total level of active agents in the filament of from 50% to about 95% by weight on a dry filament basis, wherein the weight ratio of filament-forming material to additive is 1 or less.

In one example, the filament-forming composition comprises from about 1% and/or from about 5% and/or from about 10% to about 50% and/or to about 40% and/or to about 30% and/or to about 20% by weight of the filament-forming composition of filament-forming materials; from about 1% and/or from about 5% and/or from about 10% to about 50% and/or to about 40% and/or to about 30% and/or to about 20% by weight of the filament-forming composition of active agents; and from about 20% and/or from about 25% and/or from about 30% and/or from about 40% and/or to about 80% and/or to about 70% and/or to about 60% and/or to about 50% by weight of the filament-forming composition of a volatile solvent, such as water. The filament-forming composition may comprise minor amounts of other active agents, such as less than 10% and/or less than 5% and/or less than 3% and/or less than 1% by weight of the filament-forming composition of plasticizers, pH adjusting agents, and other active agents.

The filament-forming composition is spun into one or more filaments by any suitable spinning process, such as meltblowing and/or spunbonding. In one example, the filament-forming composition is spun into a plurality of filaments by meltblowing. For example, the filament-forming composition may be pumped from an extruder to a meltblown spinnerette. Upon exiting one or more of the filament-forming holes in the spinnerette, the filament-forming composition is attenuated with air to create one or more filaments. The filaments may then be dried to remove any remaining solvent used for spinning, such as the water.

The filaments of the present invention may be collected on a belt, such as a patterned belt to form a fibrous structure comprising the filaments.

Fibrous structure

One or more, and/or a plurality of filaments of the present invention may form a fibrous structure by any suitable process known in the art. The fibrous structure may be used to deliver the active agents from the filaments of the present invention when the fibrous structure is exposed to conditions of intended use of the filaments and/or fibrous structure.

Even though the filament and/or fibrous structure of the present invention are in solid form, the filament-forming composition used to make the filaments of the present invention may be in the form of a liquid.

In one example, the fibrous structure comprises a plurality of identical or substantially identical from a compositional perspective filaments according to the present invention. In another example, the fibrous structure may comprise two or more different filaments according to the present invention. Non-limiting examples of differences in the filaments may be physical differences such as differences in diameter, length, texture, shape, rigidness, elasticity, and the like; chemical differences such as crosslinking level, solubility, melting point, Tg, active agent, filament-forming material, color, level of active agent, level of filament-forming material, presence of any coating composition on filament, biodegradable or not, hydrophobic or not, contact angle, and the like; differences in whether the filament loses its physical structure when the filament is exposed to conditions of intended use; differences in whether the filament's morphology changes when the filament is exposed to conditions of intended use; and differences in rate at which the filament releases one or more of its active agents when the filament is exposed to conditions of intended use. In one example, two or more filaments within the fibrous structure may comprise the same filament-forming material, but have different active agents. This may be the case where the different active agents may be incompatible with one another, for example an anionic surfactant (such as a shampoo active agent) and a cationic surfactant (such as a hair conditioner active agent).

In another example, as shown in Fig. 2, the fibrous structure 20 may comprise two or more different layers 22, 24 (in the z-direction of the fibrous structure 20) of filaments 10 of the present invention that form the fibrous structure

20. The filaments 10 in layer 22 may be the same as or different from the filaments 10 of layer 24. Each layer 22, 24 may comprise a plurality of identical or substantially identical or different filaments. For example, filaments that may release their active agents at a faster rate than others within the fibrous structure may be positioned to an external surface of the fibrous structure.

In another example, the fibrous structure may exhibit different regions, such as different regions of basis weight, density and/or caliper. In yet another example, the fibrous structure may comprise texture on one or more of its surfaces. A surface of the fibrous structure may comprise a pattern, such as a non-random, repeating pattern. The fibrous structure may be embossed with an emboss pattern. In another example, the fibrous structure may comprise apertures. The apertures may be arranged in a non-random, repeating pattern.

In one example, the fibrous structure may comprise discrete regions of filaments that differ from other parts of the fibrous structure.

Non-limiting examples of use of the fibrous structure of the present invention include, but are not limited to a laundry dryer substrate, washing machine substrate, washcloth, hard surface cleaning and/or polishing substrate, floor cleaning and/or polishing substrate, as a component in a battery, baby wipe, adult wipe, feminine hygiene wipe, bath tissue wipe, window cleaning substrate, oil containment and/or scavenging substrate, insect repellant substrate, swimming pool chemical substrate, food, breath freshener, deodorant, waste disposal bag, packaging film and/or wrap, wound dressing, medicine delivery, building insulation, crops and/or plant cover and/or bedding, glue substrate, skin care substrate, hair care substrate, air care substrate, water treatment substrate and/or filter, toilet bowl cleaning substrate, candy substrate, pet food, livestock bedding, teeth whitening substrates, carpet cleaning substrates, and other suitable uses of the active agents of the present invention.

The fibrous structure of the present invention may be used as is or may be coated with one or more active agents.

In another example, the fibrous structure of the present invention may be pressed into a film, for example by applying a compressive force and/or heating the fibrous structure to convert the fibrous structure into a film. The film would comprise the active agents that were present in the filaments of the present invention. The fibrous structure may be completely converted into a film or parts of the fibrous structure may remain in the film after partial conversion of the fibrous structure into the film. The films may be used for any suitable purposes that the active agents may be used for including, but not limited to the uses exemplified for the fibrous structure.

In one example, a fibrous structure having such filaments can exhibit an average disintegration time of about 60 seconds (s) or less, and/or about 30 s or less, and/or about 10 s or less, and/or about 5 s or less, and/or about 2.0 s or less, and/or 1.5 s or less as measured according to the Dissolution Test Method described herein.

In one example, a fibrous structure having such filaments can exhibit an average dissolution time of about 600 seconds (s) or less, and/or about 400 s or less, and/or about 300 s or less, and/or about 200 s or less, and/or about 175 s or less as measured according to the Dissolution Test Method described herein.

In one example, a fibrous structure having such filaments can exhibit an average disintegration time per gsm of sample of about 1.0 second/gsm (s/gsm) or less, and/or about 0.5 s/gsm or less, and/or about 0.2 s/gsm or less, and/or about 0.1 s/gsm or less, and/or about 0.05 s/gsm or less, and/or about 0.03 s/gsm or less as measured according to the Dissolution Test Method described herein.

In one example, a fibrous structure having such filaments can exhibit an average dissolution time per gsm of sample of about 10 seconds/gsm (s/gsm) or less, and/or about 5.0 s/gsm or less, and/or about 3.0 s/gsm or less, and/or about 2.0 s/gsm or less, and/or about 1.8 s/gsm or less, and/or about 1.5 s/gsm or less as measured according to the Dissolution Test Method described herein.

In one example, the fibrous structure of the present invention exhibits a thickness of greater than 0.01 mm and/or greater than 0.05 mm and/or greater than 0.1 mm and/or to about 20 mm and/or to about 10 mm and/or to about 5 mm and/or to about 2 mm and/or to about 0.5 mm and/or to about 0.3 mm as measured by the Thickness Test Method described herein.

Methods of Use

In one example, the fibrous structures of the present invention may be used to treat/clean a fabric article. For example, the method of treating a fabric article may comprise one or more steps selected from the group consisting of: (a) pre-treating the fabric article before washing the fabric article; (b) contacting the fabric article with a wash liquor formed by contacting the fibrous structure with water; (c) contacting the fabric article with the fibrous structure in a dryer; (d) drying the fabric article in the presence of the fibrous structure in a dryer; and (e) combinations thereof.

In some embodiments, the method may further comprise the step of pre-moistening the fibrous structure prior to contacting it to the fabric article to be pre-treated. For example, the fibrous structure can be pre-moistened with water and then adhered to a portion of the fabric comprising a stain that is to be pre-treated. Alternatively, the fabric may be moistened and the web placed on or adhered thereto. In some embodiments, the method may further comprise the step of selecting of only a portion of the fibrous structure for use in treating a fabric article. For example, if only one fabric

care article is to be treated, a portion of the fibrous structure may be cut and/or torn away and either placed on or adhered to the fabric or placed into water to form a relatively small amount of wash liquor which is then used to pre-treat the fabric. In this way, the user may customize the fabric treatment method according to the task at hand. In some embodiments, at least a portion of a fibrous structure may be applied to the fabric to be treated using a device. Exemplary devices include, but are not limited to, brushes and sponges. Any one or more of the aforementioned steps may be repeated to achieve the desired fabric treatment benefit.

In another example, the fibrous structures of the present invention may be used to treat/clean hair. For example, the method of treating hair may comprise the step of: contacting the hair with a wash liquor formed by contacting the fibrous structure with water.

Non-limiting Examples of Fibrous Structures

Example of Filament and Fibrous Structure Making Process

Filaments according to the present invention are produced by using a small-scale apparatus 26, a schematic representation of which is shown in Figs. 3 and 4. A pressurized tank 28 suitable for batch operations is filled with a filament-forming composition 30, for example a filament-forming composition that is suitable for making filaments useful as fabric care compositions and/or dishwashing compositions.

A pump 32 (for example a Zenith®, type PEP II pump having a capacity of 5.0 cubic centimeters per revolution (cc/rev), manufactured by Parker Hannifin Corporation, Zenith Pumps division, of Sanford, N.C., USA) is used to pump the filament-forming composition 30 to a die 34. The filament-forming composition's material flow to a die 34 is controlled by adjusting the number of revolutions per minute (rpm) of the pump 32. Pipes 36 are connected to the tank 28, the pump 32, and the die 34 in order to transport (as represented by the arrows) the filament-forming composition 30 from the tank 28 to the pump 32 and into the die 34. The die 34 as shown in Fig. 4 has two or more rows of filament-forming holes 37, which include circular extrusion nozzles 38, spaced from one another at a pitch P of about 1.524 millimeters (about 0.060 inches). The nozzles 38 have individual inner diameters of about 0.305 millimeters (about 0.012 inches) and individual outside diameters of about 0.813 millimeters (about 0.032 inches). Each individual nozzle 38 is encircled by an annular and divergently flared orifice 40 to supply attenuation air to each individual nozzle 38. The filament-forming composition 30 that is extruded through the nozzles 38 is surrounded and attenuated by generally cylindrical, humidified air streams supplied through the orifices 40 encircling the nozzles 38 to produce the filaments 10. Attenuation air is provided by heating compressed air from a source by an electrical-resistance heater, for example, a heater manufactured by Chromalox, Division of Emerson Electric, of Pittsburgh, Pa., USA. An appropriate quantity of steam is added to the attenuation air to saturate or nearly saturate the heated air at the conditions in the electrically heated, thermostatically controlled delivery pipe. Condensate is removed in an electrically heated, thermostatically controlled, separator. The filaments 10 are dried by a drying air stream having a temperature of from about 149° C. (about 300° F.) to about 315° C. (about 600° F.) by an electrical resistance heater (not shown) supplied through drying nozzles (not shown) and discharged at an angle of about 90° relative to the general orientation of the filaments 10 being spun.

The filaments are collected on a collection device to form a fibrous structure (fibrous structure) of inter-entangled filaments for example a non-random repeating pattern to a fibrous structure formed as a result of collecting the filaments on the belt or fabric.

Example 1: Fibrous Structure with Low Hydrolysis Vinyl Acetate-Vinyl Alcohol Copolymer

Table 1 below sets forth a non-limiting example of a premix (a filament-forming composition) of the present invention for making filaments and/or a fibrous structure (fibrous structure) of the present invention via the fibrous structure making process described immediately above.

Table 1

| Material | % by weight of filament-forming composition (i.e., premix) |
|---|---|
| Low $M_W$, Low hydrolysis vinyl acetate-vinyl alcohol copolymer[1] | 5.13 |
| High $M_W$, Low hydrolysis vinyl acetate-vinyl alcohol copolymer[2] | 5.13 |
| Lauryl Hydroxysultaine (40.5% activity) | 15.4 |

(continued)

| Material | % by weight of filament-forming composition (i.e., premix) |
|---|---|
| Sodium Laureth-1 Sulfate (70% activity) | 29.9 |
| Cationic cellulose (cationic polymer)[3] | 0.5 |
| Citric Acid | 0.4 |
| Distilled water | 43.54 |
| [1] PVA403, $M_W$ 30,000 g/mol, 78-82% hydrolyzed, available from Kuraray America, Inc.<br>[2] PVA420H, $M_W$ 75,000 g/mol, 78-82% hydrolyzed, available from Kuraray America, Inc.<br>[3] UCARE™ Polymer LR-400, available from Amerchol Corporation (Plaquemine, Louisiana) | |

Into an appropriately sized and cleaned vessel, the distilled water is added with stirring at 100-150 rpm. The cationic polymer (cationic cellulose) is then slowly added with constant stirring until homogenous. The low hydrolysis vinyl acetate-vinyl alcohol copolymer resin powders (PVA403 and PVA420H) are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the Sodium Laureth Sulfate and Lauryl Hydroxysultaine are added. The mixture is then heated to 75°C while continuing to stir for 45 minutes and then allowed to cool to room temperature to form the premix. This premix is then ready for spinning into filaments and ultimately making the fibrous structure therefrom.

Comparative Example A - The following comparative fibrous structure is not in accordance with the present invention and is included for comparative purposes only. Filaments and a resulting fibrous structure are made from the premix described above in Table 1 except that the total level of vinyl acetate-vinyl alcohol copolymer resin powders (PVA403 and PVA420H) is replaced with the same total level of high weight average molecular weight (100,000 g/mol), high hydrolysis vinyl acetate-vinyl alcohol copolymer resin powder (Selvol™ 523 (87-89% hydrolyzed) available from Sekisui Specialty Chemicals).

Into an appropriately sized and cleaned vessel, the distilled water is added with stirring at 100-150 rpm. The cationic polymer (cationic cellulose) is then slowly added with constant stirring until homogenous. The high weight average molecular weight, high hydrolysis vinyl acetate-vinyl alcohol copolymer resin powder (Selvol™ 523) is weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the Sodium Laureth-1 Sulfate and Lauryl Hydroxysultaine are added. The mixture is then heated to 75°C while continuing to stir for 45 minutes and then allowed to cool to room temperature to form the premix. This premix is then ready for spinning into filaments and ultimately making the fibrous structure therefrom.

Performance Comparison - For performance testing, the fibrous structure samples (Example 1 and Comparative Example A) are each cut with scissors into 1.25 gram samples on a four place balance and placed onto individual plastic weigh boats. Dimethicone with an average viscosity of 346,000 cps at 25°C (CF330M from Momentive Performance Materials, Albany, New York) is applied to each piece at a target level of 0.054 grams on a suitable four place weight balance by brushing onto the surface with a small cosmetic brush applicator. If the target weight is exceeded, the excess dimethicone is immediately removed via a small cosmetic sponge applicator. The samples are stored in bags overnight to enable the applied silicone to spread into the sample prior to testing. The dimethicone level is estimated to be approximately 4.3% by weight of the resulting sample (0.054 grams of dimethicone per 1.25 grams of sample).

The samples are tested according to the Hand Dissolution Test Method described herein to determine their dissolution rates. From Table 2 below, it is seen that while Example 1 of the present invention (using low hydrolysis vinyl acetate-vinyl alcohol copolymer) exhibits excellent dissolution, Comparative Example A (using high hydrolysis vinyl acetate-vinyl alcohol copolymer) does not.

Table 2

| | Example 1 (# of strokes) | Comparative Example A (# of strokes) |
|---|---|---|
| Dissolution Rate | 4 | >30 (gel blocking, didn't fully dissolve) |

Example 2 - Fibrous Structure with Low Hydrolysis Vinyl Acetate-Vinyl Alcohol Copolymer

Table 3 below sets forth a non-limiting example of a premix (a filament-forming composition) of the present invention for making filaments and/or a fibrous structure (fibrous structure) of the present invention via the fibrous structure making process described immediately above.

Table 3

| Components | % by weight of filament-forming composition (i.e., premix) | % by weight on a dry filament basis |
|---|---|---|
| Low $M_W$, Low hydrolysis vinyl acetate-vinyl alcohol copolymer[1] | 8.59 | 21 |
| High $M_W$, Low hydrolysis vinyl acetate-vinyl alcohol copolymer[2] | 3.68 | 9 |
| N67HSAS (82% activity) | 17.5 | 35 |
| C11.8 NaLAS (30% activity) | 47.7 | 35 |
| Distilled water | 22.53 | trace |
| [1] PVA403, $M_W$ 30,000 g/mol, 78-82% hydrolyzed, available from Kuraray America, Inc.<br>[2] PVA420H, $M_W$ 75,000 g/mol, 78-82% hydrolyzed, available from Kuraray America, Inc. | | |

Into an appropriately sized and cleaned vessel, the distilled water is added with stirring at 100-150 rpm. The cationic polymer (cationic cellulose), when present, is then slowly added with constant stirring until homogenous. The low hydrolysis PVOH (PVA403 and PVA420H) resin powders are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the C11.8 NaLAS and N67HSAS are added. The mixture is then heated to 75°C while continuing to stir for 45 minutes and then allowed to cool to room temperature. The final pH is between 7.5 - 9.0 and adjusted with citric acid or diluted sodium hydroxide if necessary. This premix is then ready for spinning into filaments and ultimately making the fibrous structure therefrom.

Comparative Example B - The following comparative fibrous structure is not in accordance with the present invention and is included for comparative purposes only. Filaments and a resulting fibrous structure are made from the premix described above in Table 3 except that the total level of vinyl acetate-vinyl alcohol copolymer resin powders (PVA403 and PVA420H) is replaced with the same total level of high weight average molecular weight (100,000 g/mol), high hydrolysis vinyl acetate-vinyl alcohol copolymer resin powder (Selvol™ 523 (87-89% hydrolyzed) available from Sekisui Specialty Chemicals).

Into an appropriately sized and cleaned vessel, the distilled water is added with stirring at 100-150 rpm. The Selvol™ 523 resin powders are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the surfactants are added. The mixture is then heated to 75°C while continuing to stir for 45 minutes and then allowed to cool to room temperature. The final pH is between 7.5 - 9.0 and adjusted with citric acid or diluted sodium hydroxide if necessary. This premix is then ready for spinning into filaments and ultimately making the fibrous structure therefrom.

Performance Comparison

The products are tested according to the Dissolution Test Method described herein to determine their dissolution rates. From Table 4, it is seen that while Example 2 of the present invention (using low hydrolysis vinyl acetate-vinyl alcohol copolymer) exhibits much better dissolution rate than comparative Example B (using high hydrolysis vinyl acetate-vinyl alcohol copolymer).

Table 4

| | Example 2 | Example B |
|---|---|---|
| Time to fully dissolve in water (15°C) | 40 seconds | 340 seconds |

Example 3 - Fibrous Structure with Low Hydrolysis Vinyl Acetate-Vinyl Alcohol Copolymer

Table 5 below sets forth a non-limiting example of a premix (a filament-forming composition) of the present invention for making filaments and/or a fibrous structure (fibrous structure) of the present invention via the fibrous structure making process described immediately above.

Table 5

| Material | % by weight of filament-forming composition (i.e., premix) | % by weight on a dry filament basis |
|---|---|---|
| C12-15 AES | 23.13 | 24.05 |
| C11.8 HLAS | 13.55 | 14.10 |
| MEA | 6.91 | 7.20 |
| N67HSAS | 3.66 | 3.82 |
| Glycerol | 2.97 | 3.09 |
| PE-20, Polyethyleneimine Ethoxylate, PEI 600 E20 | 2.81 | 3.92 |
| Ethoxylated/Propoxylated Polyethyleneimine | 2.81 | 2.92 |
| Brightener 15 | 0.25 | 0.26 |
| Amine Oxide | 1.26 | 1.32 |
| Sasol 24,9 Nonionic Surfactant | 2.17 | 2.26 |
| DTPA (Chelant) | 1.01 | 1.06 |
| Tiron (Chelant) | 1.01 | 1.05 |
| Low $M_W$, Low hydrolysis vinyl acetate - vinyl alcohol copolymer[1] | 6.90 | 16.46 |
| High $M_W$, Low hydrolysis vinyl acetate-vinyl alcohol copolymer | 6.90 | 16.46 |
| Water | 38.46 | Trace |
| [1] PVA403, $M_W$ 30,000 g/mol, 78-82% hydrolyzed, available from Kuraray America, Inc.<br>[2] PVA420H, $M_W$ 75,000 g/mol, 78-82% hydrolyzed, available from Kuraray America, Inc. | | |

Into an appropriately sized and cleaned vessel, the distilled water is added with stirring at 100-150 rpm. The cationic polymer (cationic cellulose), when present, is then slowly added with constant stirring until homogenous. The low hydrolysis vinyl acetate-vinyl alcohol copolymer resin powders (PVA403 and PVA420H) are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the surfactants and other cleaning actives are added. The mixture is then heated to 75°C while continuing to stir for 45 minutes and then allowed to cool to room temperature. The final pH is between 7.5 - 9.0 and adjusted with citric acid or diluted sodium hydroxide if necessary. This premix is then ready for spinning into filaments and ultimately making the fibrous structure therefrom.

Comparative Example C - The following comparative fibrous structure is not in accordance with the present invention and is included for comparative purposes only. Filaments and a resulting fibrous structure are made from the premix described above in Table 4 except that the total level of vinyl acetate-vinyl alcohol copolymer resin powders (PVA403 and PVA420H) is replaced with the same total level of high weight average molecular weight (100,000 g/mol), high hydrolysis vinyl acetate-vinyl alcohol copolymer resin powder (Selvol™ 523 (87-89% hydrolyzed) available from Sekisui Specialty Chemicals).

Into an appropriately sized and cleaned vessel, the distilled water is added with stirring at 100-150 rpm. The cationic polymer (cationic cellulose), when present, is then slowly added with constant stirring until homogenous. The Selvol™ 523 resin powders are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the surfactants and other cleaning actives are added. The mixture is then heated to 75°C while continuing to stir for 45 minutes and then allowed

to cool to room temperature. The final pH is between 7.5 - 9.0 and adjusted with citric acid or diluted sodium hydroxide if necessary. This premix is then ready for spinning into filaments and ultimately making the fibrous structure therefrom.

Example 4 - Fibrous Structure with Low Hydrolysis Vinyl Acetate-Vinyl Alcohol Copolymer

Table 6 below sets forth a non-limiting example of a premix (a filament-forming composition) of the present invention for making filaments and/or a fibrous structure (fibrous structure) of the present invention via the fibrous structure making process described immediately above.

Table 6

| Material | % by weight of filament-forming composition (i.e., premix) | % by weight on a dry filament basis |
|---|---|---|
| C12-15 AES | 23.13 | 24.04 |
| C11.8 HLAS | 13.55 | 14.10 |
| MEA | 6.91 | 7.20 |
| N67HSAS | 3.66 | 3.80 |
| Glycerol | 2.97 | 3.09 |
| PE-20, Polyethyleneimine Ethoxylate, PEI 600 E20 | 2.81 | 3.92 |
| Ethoxylated/Propoxylated Polyethyleneimine | 2.81 | 2.92 |
| Brightener 15 | 0.25 | 0.26 |
| Amine Oxide | 1.26 | 1.32 |
| Sasol 24,9 Nonionic Surfactant | 2.17 | 2.26 |
| DTPA (Chelant) | 1.01 | 1.06 |
| Tiron (Chelant) | 1.01 | 1.05 |
| Suds Suppressor AC8016 | 0.06 | 0.07 |
| Low $M_W$, Low hydrolysis vinyl acetate - vinyl alcohol copolymer[1] | 6.90 | 16.46 |
| High $M_W$, Low hydrolysis vinyl acetate-vinyl alcohol copolymer | 6.90 | 16.46 |
| Water | 38.46 | Trace |
| [1] PVA403, $M_W$ 30,000 g/mol, 78-82% hydrolyzed, available from Kuraray America, Inc.<br>[2] PVA420H, $M_W$ 75,000 g/mol, 78-82% hydrolyzed, available from Kuraray America, Inc. | | |

Into an appropriately sized and cleaned vessel, the distilled water is added with stirring at 100-150 rpm. The cationic polymer (cationic cellulose), when present, is then slowly added with constant stirring until homogenous. The low hydrolysis vinyl acetate-vinyl alcohol copolymer resin powders (PVA403 and PVA420H) are weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the C11.8 NaLAS and N67HSAS are added. The mixture is then heated to 75°C while continuing to stir for 45 minutes and then allowed to cool to room temperature. The final pH is between 7.5 - 9.0 and adjusted with citric acid or diluted sodium hydroxide if necessary. This premix is then ready for spinning into filaments and ultimately making the fibrous structure therefrom.

Comparative Example D - The following comparative fibrous structure is not in accordance with the present invention and is included for comparative purposes only. Filaments and a resulting fibrous structure are made from the premix described above in Table 4 except that the total level of vinyl acetate-vinyl alcohol copolymer resin powders (PVA403 and PVA420H) is replaced with the same total level of high weight average molecular weight (100,000 g/mol), high hydrolysis vinyl acetate-vinyl alcohol copolymer resin powder (Selvol™ 523 (87-89% hydrolyzed) available from Sekisui Specialty Chemicals).

Into an appropriately sized and cleaned vessel, the distilled water is added with stirring at 100-150 rpm. The

cationic polymer (cationic cellulose) is then slowly added with constant stirring until homogenous. The high weight average molecular weight, high hydrolysis vinyl acetate-vinyl alcohol copolymer resin powder (Selvol™ 523) is weighed into a suitable container and slowly added to the main mixture in small increments using a spatula while continuing to stir while avoiding the formation of visible lumps. The mixing speed is adjusted to minimize foam formation. Then the mixture is slowly heated to 75°C for 2 hours after which the surfactants and other cleaning actives are added. The mixture is then heated to 75°C while continuing to stir for 45 minutes and then allowed to cool to room temperature to form the premix. This premix is then ready for spinning into filaments and ultimately making the fibrous structure therefrom.

Test Methods

Unless otherwise specified, all tests described herein including those described under the Definitions section and the following test methods are conducted on samples that have been conditioned in a conditioned room at a temperature of 23°C ± 1.0°C and a relative humidity of 50% ± 2% for a minimum of 2 hours prior to the test. The samples tested are "usable units." "Usable units" as used herein means sheets, flats from roll stock, pre-converted flats, sheet, and/or single or multi-compartment products. All tests are conducted under the same environmental conditions and in such conditioned room. Do not test samples that have defects such as wrinkles, tears, holes, and like. Samples conditioned as described herein are considered dry samples (such as "dry filaments") for testing purposes. All instruments are calibrated according to manufacturer's specifications.

Basis Weight Test Method

Basis weight of a fibrous structure is measured on stacks of twelve usable units using a top loading analytical balance with a resolution of ± 0.001 g. The balance is protected from air drafts and other disturbances using a draft shield. A precision cutting die, measuring 3.500 in ± 0.0035 in by 3.500 in ± 0.0035 in is used to prepare all samples.

With a precision cutting die, cut the samples into squares. Combine the cut squares to form a stack twelve samples thick. Measure the mass of the sample stack and record the result to the nearest 0.001 g.

The Basis Weight is calculated in lbs/3000 $ft^2$ or $g/m^2$ as follows:

$$\text{Basis Weight} = (\text{Mass of stack}) / [(\text{Area of 1 square in stack}) \times (\text{No.of squares in stack})]$$

For example,

$$\text{Basis Weight (lbs/3000 ft}^2) = [[\text{Mass of stack (g)} / 453.6\ (\text{g/lbs})] / [12.25\ (\text{in}^2) / 144\ (\text{in}^2/\text{ft}^2) \times 12]] \times 3000$$

or,

$$\text{Basis Weight (g/m}^2) = \text{Mass of stack (g)} / [79.032\ (\text{cm}^2) / 10{,}000\ (\text{cm}^2/\text{m}^2) \times 12]$$

Report result to the nearest 0.1 lbs/3000 $ft^2$ or 0.1 $g/m^2$. Sample dimensions can be changed or varied using a similar precision cutter as mentioned above, so as at least 100 square inches of sample area in stack.

Water Content Test Method

The water (moisture) content present in a filament and/or fibrous structure is measured using the following Water Content Test Method. A filament and/or fibrous structure or portion thereof in the form of a pre-cut sheet ("sample") is placed in a conditioned room at a temperature of 23°C ± 1.0°C and a relative humidity of 50% ± 2% for at least 24 hours prior to testing. Each fibrous structure sample has an area of at least 4 square inches, but small enough in size to fit appropriately on the balance weighing plate. Under the temperature and humidity conditions mentioned above, using a balance with at least four decimal places, the weight of the sample is recorded every five minutes until a change of less than 0.5% of previous weight is detected during a 10 minute period. The final weight is recorded as the "equilibrium weight". Within 10 minutes, the samples are placed into the forced air oven on top of foil for 24 hours at 70°C ± 2°C at a relative humidity of 4% ± 2% for drying. After the 24 hours of drying, the sample is removed and weighed within 15 seconds. This weight is designated as the "dry weight" of the sample.

The water (moisture) content of the sample is calculated as follows:

$$\% \text{ Water in sample} = 100\% \times \frac{(\text{Equilibrium weight of sample} - \text{Dry weight of sample})}{\text{Dry weight of sample}}$$

The % Water (moisture) in sample for 3 replicates is averaged to give the reported % Water (moisture) in sample. Report results to the nearest 0.1%.

Hand Dissolution Test Method

A human being (tester) puts nitrile gloves on both hands. A fibrous structure sample, cut if necessary, to dimensions of approximately 43 mm x 43 mm x 4-6 mm, is placed in the palm of one of the tester's hands. 7.5 $cm^3$ of from about 30°C to about 35°C tap water is quickly applied to the sample in the palm of the hand via a syringe. The tester then places the other hand on top of the sample such that the sample is positioned between the palms of both hands. Using a circular motion, the palms of the tester's hands are rubbed together 2 strokes at a time until dissolution occurs (up to 30 strokes). The hand dissolution value is reported as the number of strokes it takes for complete dissolution (no visible fragments of the sample let in the palm of the hand) or as 30 strokes as the maximum.

Dissolution Test Method

Apparatus and Materials:

With reference to Figs. 5-7:

600 mL glass beaker 50
Magnetic Stirrer Plate 52 (Labline, Melrose Park, IL, Model No. 1250 or equivalent)
Magnetic Stirring Rod 54 (2 inch long by 3/8 inch in diameter, Teflon coated)
Thermometer (1 to 100°C +/- 1 °C)
1.25 inch paper binder clip
Alligator clamp (about one inch long) 56
Depth adjuster rod 58 and holder 60 with base 62
Timer (0-3,600 seconds accurate to at least 0.1 second)
Deionized water (equilibrated at 15°C ± 1°C)
Cutting Die -- Stainless Steel cutting die with dimensions 3.8 cm x 3.2 cm
Polaroid 35 mm Slide Mount (commercially available from Polaroid Corporation or equivalent) 35 mm Slide Mount Holder (or equivalent) 64.

Sample Preparation:

Equilibrate samples of fibrous structures in constant temperature and humidity environment of at 23°C ± 1°C and 50% ± 2% relative humidity for at least 24 hours prior to testing. The dissolution test is conducted under this temperature and relative humidity condition as well.
Measure the basis weight of the samples using Basis Weight Method defined herein.
Cut three dissolution test specimens from a fibrous structure sample to be tested using cutting die (3.8 cm x 3.2 cm), so it fits within the 35 mm slide mount 64 which has an open area dimensions 24 x 36 mm.
Lock each specimen in a separate 35 mm slide mount 64.

Equipment Setup:

As shown in Figs. 5-7, the magnetic stirring rod 54 is placed at the bottom of the 600 mL glass beaker 50 and the beaker 50 is then filled with 500 ± 5 mL of the deionized water and placed on top of a magnetic stirrer plate 52. The stirring speed is adjusted so that a steady vortex develops at the center of the beaker 50 with the vortex bottom at the 400 mL mark.
A trial run may be necessary to ensure the depth adjuster rod is set up properly. Secure the 35 mm slide mount 64 in the alligator clamp 56 of the 35 mm slide mount holder 64 such that the long end of the slide mount 64 is parallel to the water surface. The alligator clamp 56 should be positioned in the middle of the long end of the slide mount 64.

The alligator claim 56 is soldiered to the end of a depth adjuster rod 58. The depth adjuster rod 58 is set up in a way so that when the paper binder clip is lowered into the water the entire fibrous structure specimen is completely submerged in the water at the center of the beaker 50, the top of the fibrous structure specimen is at the bottom of the vortex, and the bottom of the slide mount/slide mount holder 64 is not in direct contact with the stirring bar 54. The depth adjuster rod 58 and alligator clamp 56 should be set so that the position of the fibrous structure specimen's surface is perpendicular to the flow of the water.

In one motion, drop the secured slide mount/slide mount holder 64 and alligator clamp 56 into the water and start the timer. The fibrous structure specimen is dropped so that the fibrous structure specimen is centered in the beaker. Disintegration occurs when the fibrous structure specimen breaks apart. Record this as the disintegration time. When all of the visible fibrous structure specimen is released from the slide mount 64, raise the slide mount 64 out of the water while continuing the monitor the solution for undissolved fibrous structure specimen fragments. Dissolution occurs when all fibrous structure specimen fragments are no longer visible. Record this as the dissolution time.

Three replicates of each fibrous structure specimen are run and the average disintegration and dissolution times are reported to within +/- 0.1 seconds. Average disintegration and dissolution times are in units of seconds.

The average disintegration and dissolution times are normalized for basis weight by dividing each by their respective fibrous structure specimen's basis weight as determined by the Basis Weight Method defined herein. Basis weight normalized average dissolution times are in units of seconds/gsm of sample (s/(g/m2)).

Tensile Test Method: Elongation, Tensile Strength, TEA and Modulus

Elongation, Tensile Strength, TEA and Tangent Modulus are measured on a constant rate of extension tensile tester with computer interface (a suitable instrument is the EJA Vantage from the Thwing-Albert Instrument Co. Wet Berlin, NJ) using a load cell for which the forces measured are within 10% to 90% of the limit of the cell. Both the movable (upper) and stationary (lower) pneumatic jaws are fitted with smooth stainless steel faced grips, 25.4 mm in height and wider than the width of the test specimen. An air pressure of about 60 psi is supplied to the jaws.

Eight usable units of fibrous structure are divided into two stacks of four samples each. The samples in each stack are consistently oriented with respect to machine direction (MD) and cross direction (CD). One of the stacks is designated for testing in the MD and the other for CD. Using a one inch precision cutter (Thwing Albert JDC-1-10, or similar) cut 4 MD strips from one stack, and 4 CD strips from the other, with dimensions of 1.00 in $\pm$ 0.01 in wide by 3.0 - 4.0 in long. Each strip of one usable unit thick will be treated as a unitary specimen for testing.

Program the tensile tester to perform an extension test, collecting force and extension data at an acquisition rate of 20 Hz as the crosshead raises at a rate of 2.00 in/min (5.08 cm/min) until the specimen breaks. The break sensitivity is set to 80%, i.e., the test is terminated when the measured force drops to 20% of the maximum peak force, after which the crosshead is returned to its original position.

Set the gauge length to 1.00 inch. Zero the crosshead and load cell. Insert at least 1.0 in of the unitary specimen into the upper grip, aligning it vertically within the upper and lower jaws and close the upper grips. Insert the unitary specimen into the lower grips and close. The unitary specimen should be under enough tension to eliminate any slack, but less than 5.0 g of force on the load cell. Start the tensile tester and data collection. Repeat testing in like fashion for all four CD and four MD unitary specimens.

Program the software to calculate the following from the constructed force (g) verses extension (in) curve:
Tensile Strength is the maximum peak force (g) divided by the sample width (in) and reported as g/in to the nearest 1 g/in.

Adjusted Gauge Length is calculated as the extension measured at 3.0 g of force (in) added to the original gauge length (in).

Elongation is calculated as the extension at maximum peak force (in) divided by the
Adjusted Gauge Length (in) multiplied by 100 and reported as % to the nearest 0.1%

Total Energy (TEA) is calculated as the area under the force curve integrated from zero extension to the extension at the maximum peak force (g*in), divided by the product of the adjusted Gauge Length (in) and specimen width (in) and is reported out to the nearest 1 g*in/$in^2$.

Replot the force (g) verses extension (in) curve as a force (g) verses strain curve. Strain is herein defined as the extension (in) divided by the Adjusted Gauge Length (in).

Program the software to calculate the following from the constructed force (g) verses strain curve:
Tangent Modulus is calculated as the slope of the linear line drawn between the two data points on the force (g) versus strain curve, where one of the data points used is the first data point recorded after 28 g force, and the other data point used is the first data point recorded after 48 g force. This slope is then divided by the specimen width (2.54 cm) and reported to the nearest 1 g/cm.

The Tensile Strength (g/in), Elongation (%), Total Energy (g*in/$in^2$) and Tangent Modulus (g/cm) are calculated for the four CD unitary specimens and the four MD unitary specimens. Calculate an average for each parameter separately for the CD and MD specimens.

Calculations:

Geometric Mean Tensile = Square Root of [MD Tensile Strength (g/in) x CD Tensile Strength (g/in)]

Geometric Mean Peak Elongation = Square Root of [MD Elongation (%) x CD Elongation (%)]

Geometric Mean TEA = Square Root of [MD TEA ($g*in/in^2$) x CD TEA ($g*in/in^2$)]

Geometric Mean Modulus = Square Root of [MD Modulus (g/cm) x CD Modulus (g/cm)]

Total Dry Tensile Strength (TDT) = MD Tensile Strength (g/in) + CD Tensile Strength (g/in)

Total TEA = MD TEA ($g*in/in^2$) + CD TEA ($g*in/in^2$)

Total Modulus = MD Modulus (g/cm) + CD Modulus (g/cm)

Tensile Ratio = MD Tensile Strength (g/in) / CD Tensile Strength (g/in)

Diameter Test Method

The diameter of a discrete filament or a filament within a fibrous structure is determined by using a Scanning Electron Microscope (SEM) or an Optical Microscope and an image analysis software. A magnification of 200 to 10,000 times is chosen such that the filaments are suitably enlarged for measurement. When using the SEM, the samples are sputtered with gold or a palladium compound to avoid electric charging and vibrations of the filament in the electron beam. A manual procedure for determining the filament diameters is used from the image (on monitor screen) taken with the SEM or the optical microscope. Using a mouse and a cursor tool, the edge of a randomly selected filament is sought and then measured across its width (i.e., perpendicular to filament direction at that point) to the other edge of the filament. A scaled and calibrated image analysis tool provides the scaling to get actual reading in μm. For filaments within a fibrous structure, several filament are randomly selected across the sample of the fibrous structure using the SEM or the optical microscope. At least two portions of the fibrous structure are cut and tested in this manner. Altogether at least 100 such measurements are made and then all data are recorded for statistical analysis. The recorded data are used to calculate average (mean) of the filament diameters, standard deviation of the filament diameters, and median of the filament diameters.

Another useful statistic is the calculation of the amount of the population of filaments that is below a certain upper limit. To determine this statistic, the software is programmed to count how many results of the filament diameters are below an upper limit and that count (divided by total number of data and multiplied by 100%) is reported in percent as percent below the upper limit, such as percent below 1 micrometer diameter or %-submicron, for example. We denote the measured diameter (in μm) of an individual circular filament as di.

In the case that the filaments have non-circular cross-sections, the measurement of the filament diameter is determined as and set equal to the hydraulic diameter which is four times the cross-sectional area of the filament divided by the perimeter of the cross-section of the filament (outer perimeter in case of hollow filaments). The number-average diameter, alternatively average diameter is calculated as:

$$d_{num} = \frac{\sum_{i=1}^{n} d_i}{n}$$

Thickness Test Method

Thickness of a fibrous structure is measured by cutting 5 samples of a fibrous structure sample such that each cut sample is larger in size than a load foot loading surface of a VIR Electronic Thickness Tester Model II available from Thwing-Albert Instrument Company, Philadelphia, PA. Typically, the load foot loading surface has a circular surface area of about 3.14 in2. The sample is confined between a horizontal flat surface and the load foot loading surface. The load foot loading surface applies a confining pressure to the sample of 15.5 g/$cm^2$. The thickness of each sample is the resulting gap between the flat surface and the load foot loading surface. The thickness is calculated as the average thickness of the five samples. The result is reported in millimeters (mm).

Shear Viscosity Test Method

The shear viscosity of a filament-forming composition of the present invention is measured using a capillary rheometer, Goettfert Rheograph 6000, manufactured by Goettfert USA of Rock Hill SC, USA. The measurements are conducted using a capillary die having a diameter D of 1.0 mm and a length L of 30 mm (i.e., L/D = 30). The die is attached to the lower end of the rheometer's 20 mm barrel, which is held at a die test temperature of 75 °C. A preheated to die test temperature, 60 g sample of the filament-forming composition is loaded into the barrel section of the rheometer. Rid the sample of any entrapped air. Push the sample from the barrel through the capillary die at a set of chosen rates 1,000-10,000 $seconds^{-1}$. An apparent shear viscosity can be calculated with the rheometer's software from the pressure drop the sample experiences as it goes from the barrel through the capillary die and the flow rate of the sample through the capillary die. The log (apparent shear viscosity) can be plotted against log (shear rate) and the plot can be fitted by the power law, according to the formula $\eta = \mathbf{K}\gamma^{\mathbf{n-1}}$**,** wherein **K** is the material's viscosity constant, **n** is the material's thinning index and **y** is the shear rate. The reported apparent shear viscosity of the filament-forming composition herein is calculated from an interpolation to a shear rate of 3,000 $sec^{-1}$ using the power law relation.

Weight Average Molecular Weight

The weight average molecular weight (Mw) of a material, such as a polymer, is determined by Gel Permeation Chromatography (GPC) using a mixed bed column. A high performance liquid chromatograph (HPLC) having the following components: Millenium®, Model 600E pump, system controller and controller software Version 3.2, Model 717 Plus autosampler and CHM-009246 column heater, all manufactured by Waters Corporation of Milford, MA, USA, is utilized. The column is a PL gel 20 μm Mixed A column (gel molecular weight ranges from 1,000 g/mol to 40,000,000 g/mol) having a length of 600 mm and an internal diameter of 7.5 mm and the guard column is a PL gel 20 μm, 50 mm length, 7.5 mm ID. The column temperature is 55°C and the injection volume is 200 μL. The detector is a DAWN® Enhanced Optical System (EOS) including Astra® software, Version 4.73.04 detector software, manufactured by Wyatt Technology of Santa Barbara, CA, USA, laser-light scattering detector with K5 cell and 690 nm laser. Gain on odd numbered detectors set at 101. Gain on even numbered detectors set to 20.9. Wyatt Technology's Optilab® differential refractometer set at 50°C. Gain set at 10. The mobile phase is HPLC grade dimethylsulfoxide with 0.1% w/v LiBr and the mobile phase flow rate is 1 mL/min, isocratic. The run time is 30 minutes.

A sample is prepared by dissolving the material in the mobile phase at nominally 3 mg of material /1 mL of mobile phase. The sample is capped and then stirred for about 5 minutes using a magnetic stirrer. The sample is then placed in an 85°C convection oven for 60 minutes. The sample is then allowed to cool undisturbed to room temperature. The sample is then filtered through a 5μm Nylon membrane, type Spartan-25, manufactured by Schleicher & Schuell, of Keene, NH, USA, into a 5 milliliter (mL) autosampler vial using a 5 mL syringe.

For each series of samples measured (3 or more samples of a material), a blank sample of solvent is injected onto the column. Then a check sample is prepared in a manner similar to that related to the samples described above. The check sample comprises 2 mg/mL of pullulan (Polymer Laboratories) having a weight average molecular weight of 47,300 g/mol. The check sample is analyzed prior to analyzing each set of samples. Tests on the blank sample, check sample, and material test samples are run in duplicate. The final run is a run of the blank sample. The light scattering detector and differential refractometer is run in accordance with the "Dawn EOS Light Scattering Instrument Hardware Manual" and "Optilab® DSP Interferometric Refractometer Hardware Manual," both manufactured by Wyatt Technology Corp., of Santa Barbara, CA, USA, and both incorporated herein by reference.

The weight average molecular weight of the sample is calculated using the detector software. A dn/dc (differential

change of refractive index with concentration) value of 0.066 is used. The baselines for laser light detectors and the refractive index detector are corrected to remove the contributions from the detector dark current and solvent scattering. If a laser light detector signal is saturated or shows excessive noise, it is not used in the calculation of the molecular mass. The regions for the molecular weight characterization are selected such that both the signals for the 90° detector for the laser-light scattering and refractive index are greater than 3 times their respective baseline noise levels. Typically the high molecular weight side of the chromatogram is limited by the refractive index signal and the low molecular weight side is limited by the laser light signal.

The weight average molecular weight can be calculated using a "first order Zimm plot" as defined in the detector software. If the weight average molecular weight of the sample is greater than 1,000,000 g/mol, both the first and second order Zimm plots are calculated, and the result with the least error from a regression fit is used to calculate the molecular mass. The reported weight average molecular weight is the average of the two runs of the material test sample.

Filament Composition Test Method

In order to prepare filaments for filament composition measurement, the filaments must be conditioned by removing any coating compositions and/or materials present on the external surfaces of the filaments that are removable. An example of a method for doing so is washing the filaments 3 times with a suitable solvent that will remove the external coating composition while leaving the filaments unaltered. The filaments are then air dried at 23°C ± 1.0°C until the filaments comprise less than 10% moisture. A chemical analysis of the conditioned filaments is then completed to determine the compositional make-up of the filaments with respect to the filament-forming materials and the active agents and the level of the filament-forming materials and active agents present in the filaments.

The compositional make-up of the filaments with respect to the filament-forming material and the active agents can also be determined by completing a cross-section analysis using TOF-SIMs or SEM. Still another method for determining compositional make-up of the filaments uses a fluorescent dye as a marker. In addition, as always, a manufacturer of filaments should know the compositions of their filaments.

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in such cited document, the meaning or definition assigned to that term in this document shall govern.

While particular embodiments of the present invention have been illustrated and described, it would be obvious to those skilled in the art that various other changes and modifications can be made without departing from the scope of the appended claims.

## Claims

1. A filament comprising one or more filament-forming materials comprising one or more vinyl acetate-vinyl alcohol copolymers wherein at least one of the vinyl acetate-vinyl alcohol copolymers comprises 84 mol% or less alcohol units; and
   wherein the filament comprises one or more active agents present within the filament, wherein the least one of active agent is selected from the group consisting of: skin benefit agents, medicinal agents, lotions, fabric care agents, dishwashing agents, carpet care agents, surface care agents, hair care agents, air care agents, and mixtures thereof; and wherein the one or more filament-forming materials and the one or more active agents are present in the filament at a weight ratio of filament-forming materials to active agents of 1.85 or less.

2. The filament according to Claim 1 wherein at least one of the active agents are releasable from the filament when exposed to conditions of intended use.

3. The filament according to Claim 1 or 2 wherein at least one of the active agents comprises a surfactant, preferably wherein the surfactant is selected from the group consisting of: anionic surfactants, cationic surfactants, nonionic surfactants, zwitterionic surfactants, and mixtures thereof.

4. The filament according to any of the preceding claims wherein the filament-forming materials further comprise a polymer selected from the group consisting of: pullulan, hydroxypropylmethyl cellulose, hydroxyethyl cellulose, hydroxypropyl cellulose, polyvinyl pyrrolidone, carboxymethyl cellulose, sodium alginate, xanthan gum, tragacanth gum, guar gum, acacia gum, Arabic gum, polyacrylic acid, methylmethacrylate copolymer, carboxyvinyl polymer, dextrin, pectin, chitin, levan, elsinan, collagen, gelatin, zein, gluten, soy protein, casein, starch, starch derivatives,

hemicellulose, hemicellulose derivatives, proteins, chitosan, chitosan derivatives, polyethylene glycol, tetramethylene ether glycol, hydroxymethyl cellulose, and mixtures thereof.

**5.** The filament according to any of the preceding claims wherein the filament comprises two or more different active agents.

**6.** The filament according to any of the preceding claims wherein the filament further comprises one or more of the following:

a. a dissolution aid;
b. an extensional aid;
c. a plasticizer, preferably wherein the plasticizer is selected from the group consisting of: glycerin, ethylene glycol, polyethylene glycol, propylene glycol, glycidol, urea, sorbitol, xylitol, maltitol, sugars, ethylene bisformamide, amino acids, and mixtures thereof; and
d. mixtures thereof.

**7.** The filament according to any of the preceding claims wherein the filament exhibits a water content of from 0% to 20% by weight as measured according to the Water Content Test Method.

**8.** The filament according to any of the preceding claims wherein the total level of the one or more filament-forming materials present in the filament is 90% or less by weight on a dry filament basis and the total level of the one or more active agents present in the filament is greater than 10% or greater by weight on a dry filament basis.

**9.** The filament according to any of the preceding claims wherein the filament exhibits a diameter of less than 50 μm as measured according to the Diameter Test Method.

**10.** The filament according to any of the preceding claims wherein the filament further comprises a coating composition present on an external surface of the fibrous structure, preferably wherein the coating composition comprises one or more active agents.

**11.** A fibrous structure comprising one or more filaments according to any of the preceding claims.

**12.** The fibrous structure according to Claim 11 wherein the fibrous structure exhibits one or more of the following properties:

a. a Hand Dissolution of less than 30 strokes as measured according to the Hand Dissolution Test Method;
b. an average disintegration time of 60 s or less as measured according to the Dissolution Test Method;
c. an average dissolution time of 600 s or less as measured according to the Dissolution Test Method;
d. an average basis weight normalized disintegration time of 1.0 s/gsm or less as measured according to the Dissolution Test Method;
e. an average basis weight normalized dissolution time of 10 s/gsm or less as measured according to the Dissolution Test Method; and
f. combinations thereof.

**13.** The fibrous structure according to Claim 11 or 12 wherein the fibrous structure comprises a coating composition present on an external surface of the fibrous structure, preferably wherein the coating composition comprises one or more active agents.

## Patentansprüche

**1.** Faden, der ein oder mehrere fadenbildende Materialien umfasst, die ein oder mehrere Vinylacetat-Vinylalkohol-Copolymere umfassen, wobei mindestens eines der Vinylacetat-Vinylalkohol-Copolymere zu 84 Mol-% oder weniger Alkoholeinheiten umfasst; und
wobei der Faden einen oder mehrere Wirkstoffe umfasst, die innerhalb des Fadens vorhanden sind, wobei der mindestens eine Wirkstoff ausgewählt ist aus der Gruppe bestehend aus: Hautpflegemitteln, medizinischen Wirkstoffen, Lotionen, Textilpflegemitteln, Geschirrspülmitteln, Teppichpflegemitteln, Oberflächenpflegemitteln, Haarpflegemitteln, Luftpflegemitteln und Mischungen davon; und wobei das eine oder mehreren fadenbildenden Materialien und der eine oder die mehreren Wirkstoffe in dem Faden in einem Gewichtsverhältnis von fadenbildenden Materialien zu Wirkstoffen von 1,85 oder weniger vorhanden sind.

2. Faden nach Anspruch 1, wobei mindestens einer der Wirkstoffe von dem Faden freigesetzt werden kann, wenn dieser den Bedingungen des bestimmungsgemäßen Gebrauchs ausgesetzt wird.

3. Faden nach Anspruch 1 oder 2, wobei mindestens einer der Wirkstoffe ein Tensid umfasst, wobei das Tensid vorzugsweise ausgewählt ist aus der Gruppe bestehend aus: anionischen Tensiden, kationischen Tensiden, nichtionischen Tensiden, zwitterionischen Tensiden und Mischungen davon.

4. Faden nach einem der vorstehenden Ansprüche, wobei die fadenbildenden Materialien ferner ein Polymer umfassen, ausgewählt aus der Gruppe bestehend aus: Pullulan, Hydroxypropylmethylcellulose, Hydroxyethylcellulose, Hydroxypropylcellulose, Polyvinylpyrrolidon, Carboxymethylcellulose, Natriumalginat, Xanthangummi, Tragantgummi, Guargummi, Akaziengummi, Gummiarabikum, Polyacrylsäure, Methylmethacrylat-Copolymer, Carboxyvinylpolymer, Dextrin, Pektin, Chitin, Levan, Elsinan, Kollagen, Gelatine, Zein, Gluten, Sojaprotein, Casein, Stärke, Stärkederivaten, Hemicellulose, Hemicellulosederivaten, Proteinen, Chitosan, Chitosanderivaten, Polyethylenglycol, Tetramethylenetherglycol, Hydroxymethylcellulose und Mischungen davon.

5. Faden nach einem der vorstehenden Ansprüche, wobei der Faden zwei oder mehrere verschiedene Wirkstoffe umfasst.

6. Faden nach einem der vorstehenden Ansprüche, wobei der Faden ferner eines der mehrere aus Folgendem umfasst:

a. ein Hilfsmittel für die Zersetzung;
b. ein Streckmittel;
c. einen Weichmacher, wobei der Weichmacher vorzugsweise ausgewählt ist aus der Gruppe bestehend aus: Glycerin, Ethylenglycol, Polyethylenglycol, Propylenglycol, Glycidol, Harnstoff, Sorbit, Xylit, Maltit, Zuckern, Ethylenbisformamid, Aminosäuren und Mischungen davon; und
d. Mischungen davon.

7. Faden nach einem der vorstehenden Ansprüche, wobei der Faden einen Wassergehalt von 0 Gew.-% bis 20 Gew.-% gemäß Messung nach dem Wassergehalt-Prüfverfahren aufweist.

8. Faden nach einem der vorstehenden Ansprüche, wobei die Gesamtkonzentration des einen oder der mehreren fadenbildenden Materialien, die in dem Faden vorhanden sind, auf einer Trockenfilamentbasis 90 Gew.-% oder weniger beträgt und die Gesamtkonzentration des einen oder der mehreren Wirkstoffe, die in dem Faden vorhanden sind, auf einer Trockenfilamentbasis mehr als 10 Gew.-% oder mehr beträgt.

9. Faden nach einem der vorstehenden Ansprüche, wobei der Faden einen Durchmesser von weniger als 50 µm gemäß Messung nach dem Durchmesserprüfverfahren aufweist.

10. Faden nach einem der vorstehenden Ansprüche, wobei der Faden ferner eine auf einer Außenfläche der Faserstruktur vorhandene Beschichtungszusammensetzung umfasst, wobei vorzugsweise die Beschichtungszusammensetzung einen oder mehrere Wirkstoffe umfasst.

11. Faserstruktur, die einen oder mehrere Fäden nach einem der vorstehenden Ansprüche umfasst.

12. Faserstruktur nach Anspruch 11, wobei die Faserstruktur eine oder mehrere der folgenden Eigenschaften aufweist:

a. eine Handauflösung von weniger als 30 Handschlägen gemäß Messung nach dem Handauflöseprüfverfahren;
b. eine durchschnittliche Zerfallszeit von 60 s oder weniger gemäß Messung nach dem Auflöseprüfverfahren;
c. eine durchschnittliche Auflösezeit von 600 s oder weniger gemäß Messung nach dem Auflöseprüfverfahren;
d. eine durchschnittliche flächengewichtsnormalisierte Zerfallszeit von 1,0 s/gsm oder weniger gemäß Messung nach dem Auflöseprüfverfahren;
e. an durchschnittliche flächengewichtsnormalisierte Auflösezeit von 10 s/gsm oder weniger gemäß Messung nach dem Auflöseprüfverfahren; und
f. Kombinationen davon.

**13.** Faserstruktur nach Anspruch 11 oder 12, wobei die Faserstruktur eine auf einer Außenfläche der Faserstruktur vorhandene Beschichtungszusammensetzung aufweist, wobei vorzugsweise die Beschichtungszusammensetzung einen oder mehrere Wirkstoffe umfasst.

## Revendications

**1.** Filament comprenant un ou plusieurs matériaux formant filament comprenant un ou plusieurs copolymères d'acétate de vinyle-alcool vinylique dans lequel au moins l'un des copolymères d'acétate de vinyle-alcool vinylique comprend 84 % molaires ou moins de motifs alcool ; et
où le filament comprend un ou plusieurs agents actifs présents au sein du filament, dans lequel le ou les agents actifs sont choisis dans le groupe constitué de : agents de soin cutané, agents médicinaux, lotions, agents pour le soin des tissus, agents pour le lavage de la vaisselle, agents pour le soin des tapis, agents pour le soin des surfaces, agents de soin capillaire, agents pour le soin de l'air, et leurs mélanges ; et dans lequel le ou les matériaux formant filament et le ou les agents actifs sont présents dans le filament à un rapport pondéral des matériaux formant filament aux agents actifs de 1,85 ou moins.

**2.** Filament selon la revendication 1, dans lequel au moins l'un des agents actifs est libérable du filament lorsqu'il est exposé aux conditions d'utilisation prévue.

**3.** Filament selon la revendication 1 ou 2, dans lequel au moins l'un des agents actifs comprend un agent tensioactif, de préférence dans lequel l'agent tensioactif est choisi dans le groupe constitué de : agents tensioactifs anioniques, agents tensioactifs cationiques, agents tensioactifs non ioniques, agents tensioactifs zwittérioniques, et leurs mélanges.

**4.** Filament selon l'une quelconque des revendications précédentes, dans lequel les matériaux formant filament comprennent en outre un polymère choisi dans le groupe constitué de : pullulane, hydroxypropylméthyl-cellulose, hydroxyéthylcellulose, hydroxypropylcellulose, polyvinylpyrrolidone, carboxyméthylcellulose, alginate de sodium, gomme de xanthane, gomme de tragacanthe, gomme de guar, gomme d'acacia, gomme arabique, acide polyacrylique, copolymère de méthacrylate de méthyle, polymère carboxyvinyle, dextrine, pectine, chitine, lévane, elsinane, collagène, gélatine, zéine, gluten, protéine de soja, caséine, amidon, dérivés d'amidon, hémicellulose, dérivés d'hémicellulose, protéines, chitosan, dérivés de chitosan, polyéthylène glycol, tétraméthylène éther glycol, hydroxyméthylcellulose, et leurs mélanges.

**5.** Filament selon l'une quelconque des revendications précédentes, où le filament comprend deux agents actifs différents ou plus.

**6.** Filament selon l'une quelconque des revendications précédentes, où le filament comprend en outre une ou plusieurs parmi les substances suivantes :

a. un auxiliaire de dissolution ;
b. un auxiliaire d'extension ;
c. un plastifiant, de préférence dans lequel le plastifiant est choisi dans le groupe constitué de : glycérine, éthylène glycol, polyéthylène glycol, propylène glycol, glycidol, urée, sorbitol, xylitol, maltitol, sucres, éthylène bisformamide, acides aminés, et leurs mélanges ; et
d. leurs mélanges.

**7.** Filament selon l'une quelconque des revendications précédentes, où le filament présente une teneur en eau allant de 0 % à 20 % en poids telle que mesurée selon le procédé de test de teneur en eau.

**8.** Filament selon l'une quelconque des revendications précédentes, dans lequel le taux total du ou des matériaux formant filament présents dans le filament est de 90 % ou moins en poids sur une base de filament sec et le taux total du ou des agents actifs présents dans le filament est supérieur à 10 % ou plus en poids sur une base de filament sec.

**9.** Filament selon l'une quelconque des revendications précédentes, où le filament présente un diamètre inférieur à 50 $\mu$m tel que mesuré selon le procédé de test de diamètre.

**10.** Filament selon l'une quelconque des revendications précédentes, où le filament comprend en outre une composition de revêtement présente sur une surface externe de la structure fibreuse, de préférence dans lequel la composition de revêtement comprend un ou plusieurs agents actifs.

**11.** Structure fibreuse comprenant un ou plusieurs filaments selon l'une quelconque des revendications précédentes.

**12.** Structure fibreuse selon la revendication 11, où la structure fibreuse présente une ou plusieurs des propriétés suivantes :

a. une dissolution à la main inférieure à 30 passages telle que mesurée selon le procédé de test de dissolution à la main ;
b. un temps moyen de désintégration de 60 s ou moins tel que mesuré selon le procédé de test de dissolution ;
c. un temps moyen de dissolution de 600 s ou moins tel que mesuré selon le procédé de test de dissolution ;
d. un temps de désintégration moyen normalisé à la masse surfacique de 1,0 $s/g/m^2$ ou moins tel que mesuré selon le procédé de test de dissolution ;
e. un temps de dissolution moyen normalisé à la masse surfacique de 10 $s/g/m^2$ ou moins tel que mesuré selon le procédé de test de dissolution ; et
f. leurs combinaisons.

**13.** Structure fibreuse selon la revendication 11 ou 12, où la structure fibreuse comprend une composition de revêtement présente sur une surface externe de la structure fibreuse, de préférence dans laquelle la composition de revêtement comprend un ou plusieurs agents actifs.

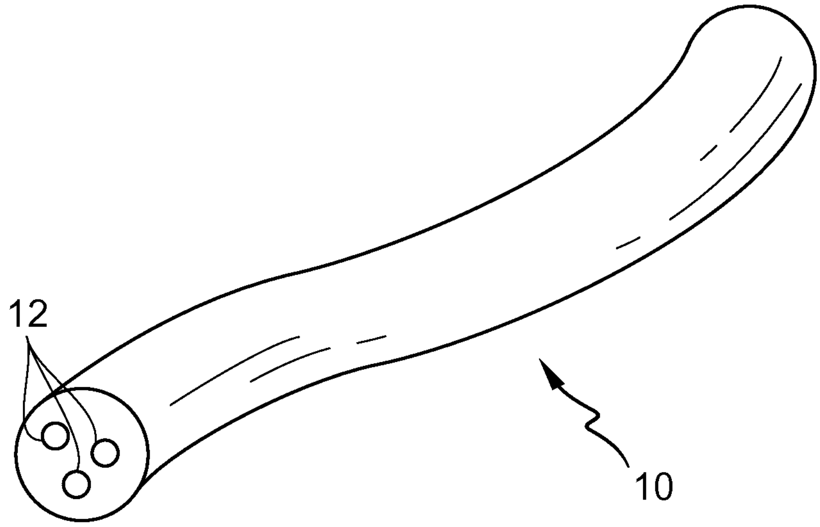


Fig. 1

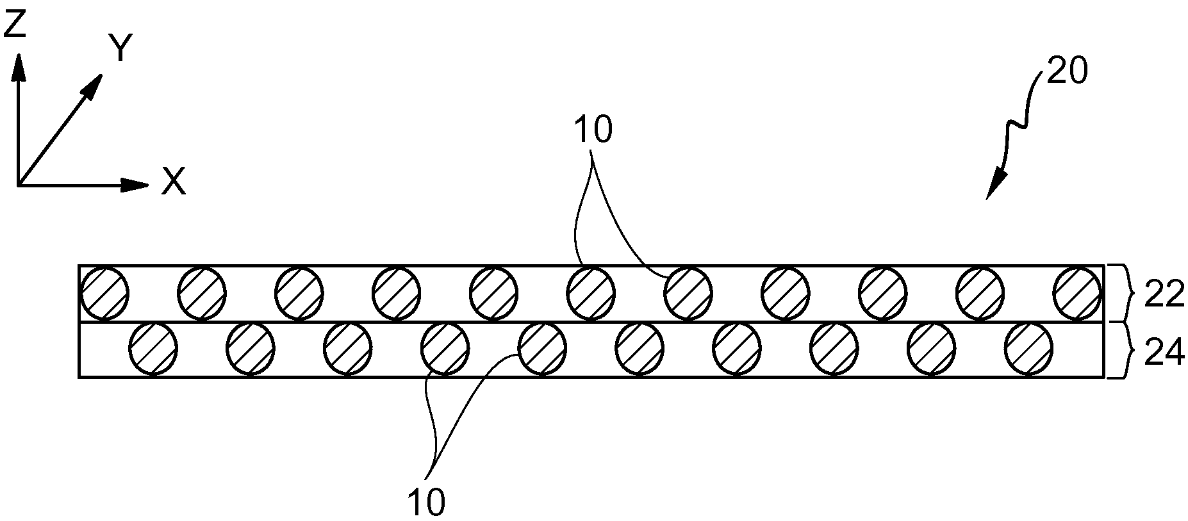


Fig. 2

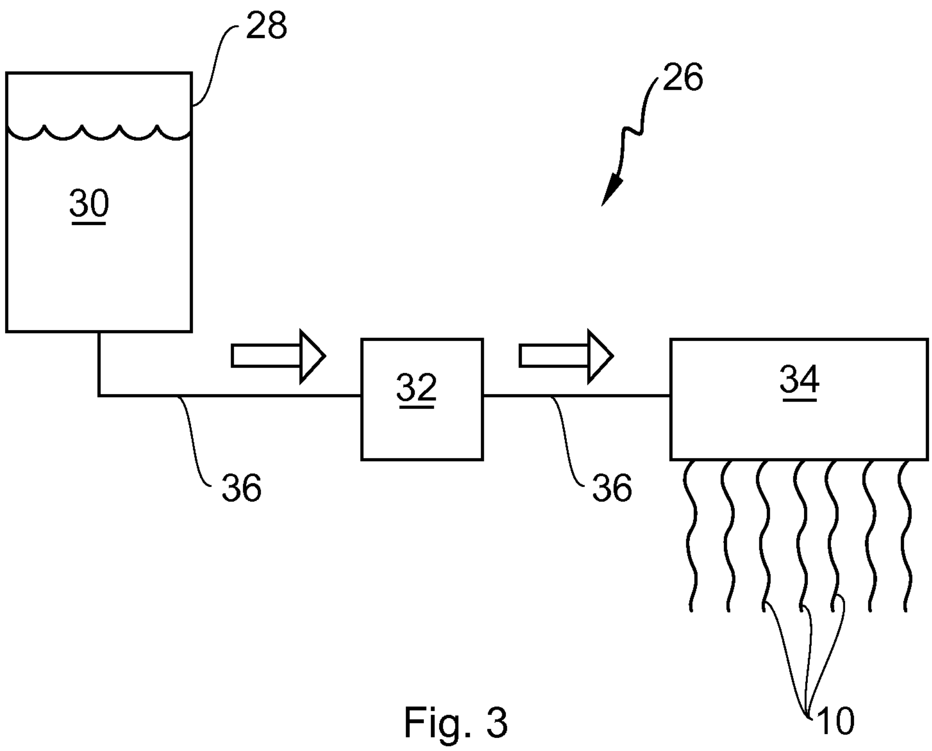


Fig. 3

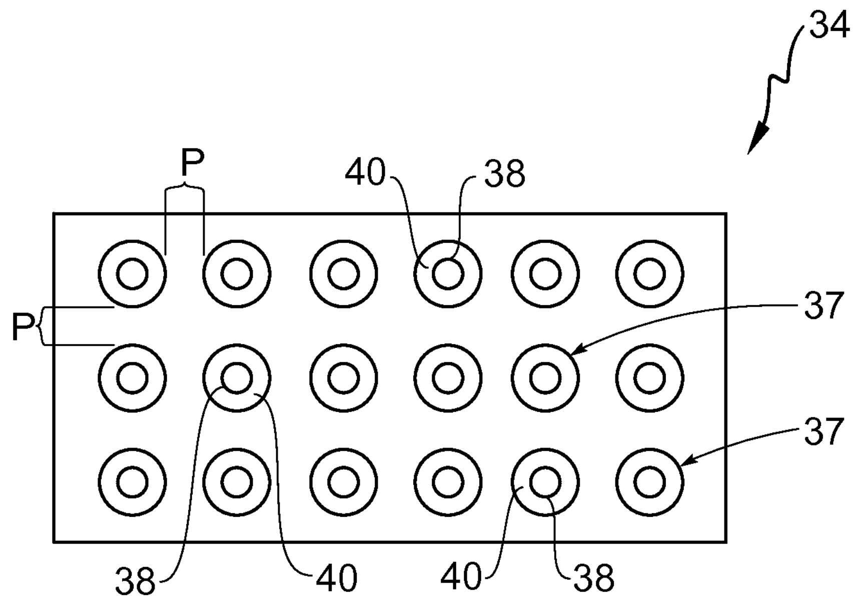


Fig. 4

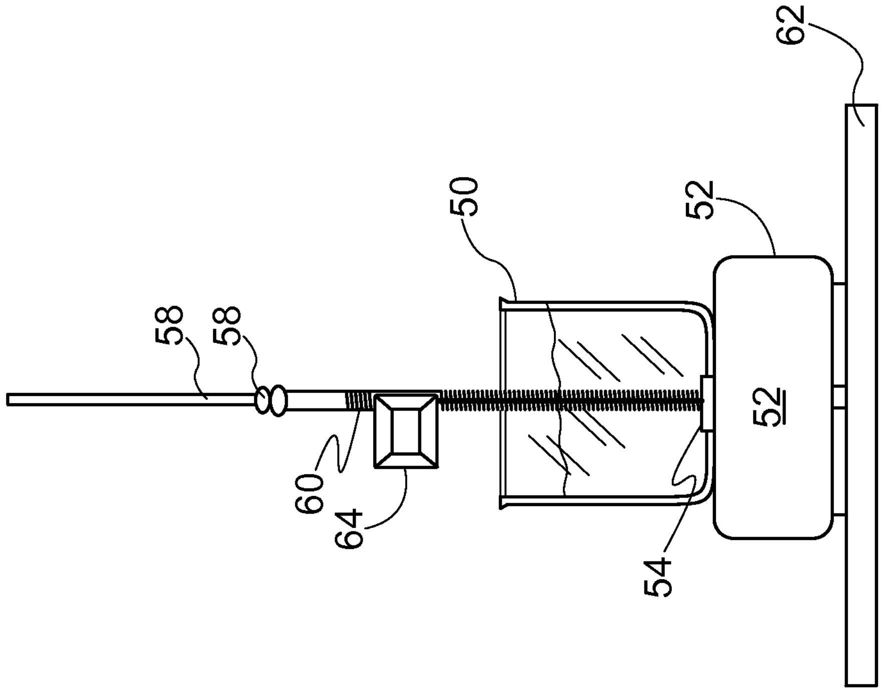


Fig. 7

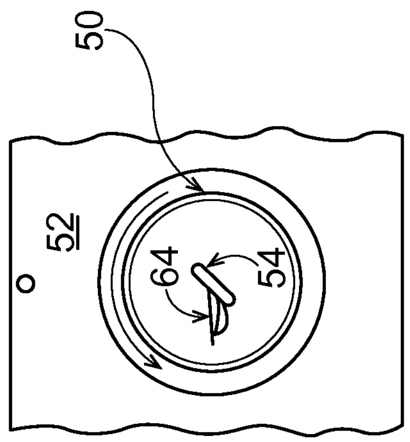


Fig. 6

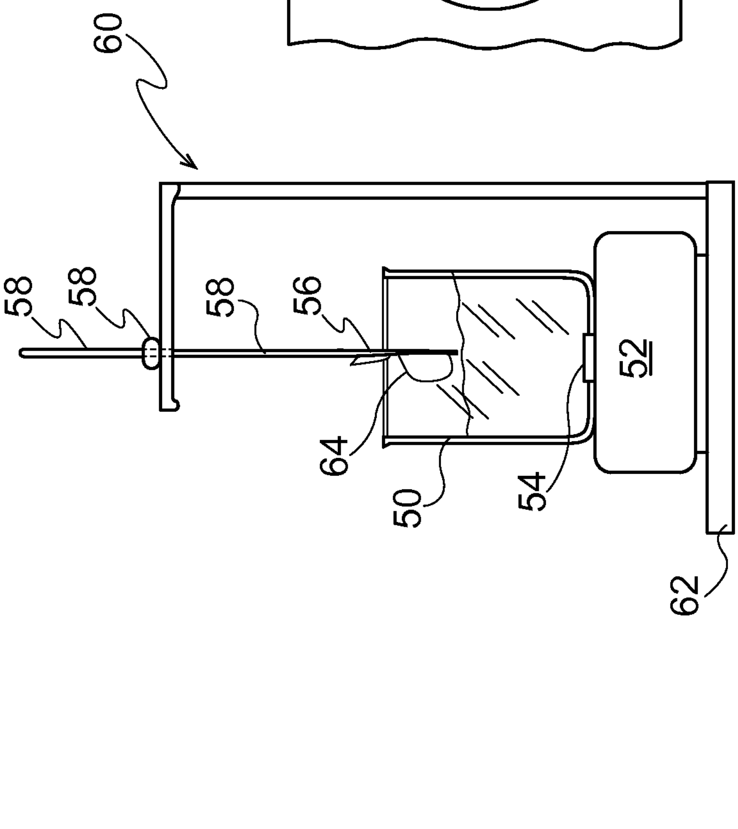


Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8466099 B
- US 8461090 B
- US 20120052036 A
- US 2486921 A
- US 2486922 A
- US 2396278 A
- US 5104646 A
- US 5106609 A
- US 3929678 A
- US 2658072 A
- US 2438091 A
- US 2528378 A


### Non-patent literature cited in the description


- Polyvinyl Compounds, Others. Ullmann's Encyclopedia of Industrial Chemistry. 2000, vol. 29, 605-609
- CTFA Cosmetic Ingredient Handbook. The Cosmetic, Toiletries, and Fragrance Association, Inc, 1988
- McCutcheon's, Emulsifiers and Detergents. M. C. Publishing Co, 1989
- McCutcheon's Detergents and Emulsifiers. Allured Publishing Corp, 2010
- McCutcheon's Functional Materials. 2010